# EUROPEAN PATENT SPECIFICATION

(11) **EP 3 831 358 B1**
(45) Date of publication and mention of the grant of the patent: **12.03.2025**
(21) Application number: 19383075.9
(22) Date of filing: 04.12.2019
(51) Int. Cl.: C07K 14/54, A61K 9/00, A61Q 19/08, A61K 8/64, A61K 8/06, A61K 8/04, A61K 9/08, A61K 9/10, A61K 38/00

(54) **PEPTIDES AND COMPOSITIONS FOR USE IN COSMETICS AND MEDICINE**
PEPTIDE UND ZUSAMMENSETZUNGEN ZUR VERWENDUNG IN KOSMETIKA UND ARZNEIMITTELN
PEPTIDES ET COMPOSITIONS À UTILISER EN COSMÉTIQUE ET EN MÉDECINE

(43) Date of publication of application: 09.06.2021
(73) Proprietor: Lipotrue, S.L., 08850 Gava (ES)
(72) Inventor: Grau-Campistany, Ariadna, 08028 Barcelona. (ES); Pastor, Silvia, 03540 Alicante (ES); Carulla, Patricia, 08024 Barcelona (ES); Escudero, Juan Carlos, 08021 Barcelona (ES)
(74) Representative: Clarke, Modet y Cía., S.L.

(56) References cited:
- EP-A1- 2 740 484
- JP-A- 2011 098 900
- US-A- 5 958 752

## Description

The present invention relates to the field of molecular biology, more precisely to molecular biology applied to cosmetics and/or medicine, even more precisely to peptides and compositions comprising said peptides, able to reduce, prevent and/or eliminate signs of skin aging, for skin rejuvenation and/or to reduce, prevent and/or eliminate skin imperfections, more precisely in the eyes. More preferably, the present invention relates to peptides and compositions comprising them which are useful for eye care and/or to treat conditions related with the eye.

In the last decades there is an increased concern in the population regarding personal aesthetics and to try to delay or minimize the appearance of signs of skin aging.

The skin is the largest organ in humans and due to its location, in the body interface, is subject to intrinsic (chronologic) aging and extrinsic aging, the latter being caused by environmental factors (such as, for example, UV radiation, smoking, pollution or sleep deprivation).

In this sense, some of the most common skin aging signs are located in the face (facial skin aging signs), more precisely, in the surroundings of the eyes, for example, eyebags, eyelid imperfections, wrinkles in the surrounding of the eyes and dark circles (periorbital hyperchromia). These are also amongst the skin aging signs which cause more concern in the society as their presence and/or increase provide for an older appearance. Therefore, there is an interest in the prevention and/or treatment of all these signs.

The most important eyelid imperfections are those associated with droopy and/or sagging eyelids. They are normally due to the fact that the skin and muscles around your eyes get weaker with ageing causing the eyelid to sag. Exposure to the sun and the natural effects of ageing can cause the skin around your eyes to sag more. Regarding ageing, as the skin becomes less elastic with age, and the face becomes less plump and firm, the result is often loose or excess skin around the eyes. Sagging eyelids can be due to different reasons or underlying medical or cosmetic disorders. Normally, sagging eyelids are due to dermatochalasis, ptosis (for example, aponeurotic ptosis and aponeurotic blepharoptosis), ectropion and entropion (Wendell Damasceno, R. et al. Eyelid aging: pathophysiology and clinical magement, Arq Bras Oftalmol. 2015; 78(5):328-31). Eyelid imperfections (preferably sagging eyelids) are normally considered a cosmetic condition but, in certain situations (for example, extensive sagging eyelid), they can lead to visual field loss, ocular or eyelid irritation and headaches, being, thus, a medical condition.

As it is well known in the state of the art most of the skin aging signs are produced or originated due to oxidative stress and/or inflammation, which are in turn triggered by chronological aging or by environmental agents (for example, UV radiation or sleep deprivation).

Interleukin-33 (hereinafter, IL-33) is a member of the IL-1 family. It is an inflammation-induced factor with a dual function exercising its role as an intracellular regulator of gene expression, as well as, an extracellular alarm mediator. It is a ligand for ST2, a heterodimeric membrane-bound receptor of the orphan IL-1 family receptor.

IL-33 is expressed by a wide variety of cell types, including fibroblasts, mast cells, dendritic cells, macrophages, osteoblasts, endothelial cells, and epithelial cells (such as keratinocytes) (Martin, N.T. and Martin, M.U. Interleukin 33 is a guardian of barriers and a local alarmin, Nature Immunology (2016) 17, 122-131).

During homeostasis, nuclear IL-33 is constitutively expressed to high levels in epithelial barrier tissues, such as lung, skin and stomach. Full length bioactive IL-33 is released extracellularly from the nucleus upon tissue damage and cell death (or cellular stress), following exposure to allergens or infection with viruses or parasites. After release, IL-33 'raises the alarm' in the immune system by activating various types of immune cells, including mast cells and, most importantly, ILC2s, which secrete large amounts of IL-5 and IL-13. After programmed cell death (apoptosis), IL-33 is inactivated by caspases to avoid alerting the immune system unnecessarily. Although full length IL-33 is active, it can be processed by inflammatory proteases (cathepsin G, elastase) into shorter 'hyperactive' mature forms, which may be the crucial bioactive forms in vivo.

The range and duration of the action of IL-33 is regulated by its rapid oxidation that inhibits its binding to ST2.

In the recent years IL-33 has gained attention due to the great number of signaling processes in which it has revealed to participate and, hence, the key role that it seems to have in a number of cosmetic and medical features, for example:
- Skin inflammation: Exposure to UV radiation induces IL-33 expression in keratinocytes and dermal fibroblasts (Napier Byrne, S., et al. The immune-modulating cytokine and endogenous alarmin Interleukin-33 is upregulated in skin exposed to inflammatory UVB Radiation. The American Journal of Pathology, 179 (2011), 211-222).
- Skin barrier: IL-33 has a skin barrier modulation effect. An increase in IL-33 produces a decrease in the expression of filaggrin and disturbs the skin barrier facilitating the entrance of allergens, bacteria and virus (Seltmann, J. et.al. IL33 impacts on the skin barrier by downregulating the expression of filaggrin. J Allergy Clin Immunol, Volume 135, Number 6, pages 1659-1661).
- Vascularity: IL-33 promotes angiogenesis and vascular permeability (Choi, Y. et.al. Interleukin-33 induces angiogenesis and vascular permeability through ST2/TRAF6-mediated endothelial nitric oxide production. Blood, 1 October 2009, volume 114, number 14, pages 3117-3126).
- Pigmentation: IL-33 has a pro-melanogenic activity, by improving melanin biosynthesis in melanocytes (Zhou, J. et.al. Enhancement of the p38 MAPK and PKA signaling pathways is associated with the pro-melanogenic activity of Interleukin 33 in primary melanocytes. Journal of Dermatological Science, 73 (2014), 110-116).

Therefore, IL-33 produces a decrease in keratinocytes cohesion, an increase in pigmentation and an increase in inflammation and vascular permeability and, hence, is related with all the skin signs mentioned above.

Sleep deprivation, (this is, a shorter than optimal sleep time, a complete lack of sleeping period or changes in the circadian patterns) has also an important impact in the skin and is a promoter of skin aging and/or skin imperfections. Some skin attributes associated with sleep deficiency include rough, dull and dry skin as well as droopy eyelids and dark eye circles. The effect is not limited to visual characteristics, but sleep deprivation can affect the health and physiology of the skin, such as exacerbating skin disorders, impairing the integrity of the skin barrier, increasing the production of inflammatory cytokines, glucocorticoids as well as increasing glucose and cortisol levels and decreasing melatonin levels (Kim, M.A. et al. The effect of sleep deprivation on the biophysical properties of facial skin, Journal of cosmetics, dermatological sciences and application (2017) 7, 34-47; and Guan L., Mehra R., Baron E. (2017) Sleep and Aging Skin. In: Farage M., Miller K., Maibach H. (eds) Textbook of Aging Skin. Springer, Berlin, Heidelberg).

The elevation of glucose concentration negatively impacts the proliferation of keratinocytes as well as fibroblasts of the skin (Kruse, C.R. et al. The effect of local hyperglucemia on skin cells in vitro and on wound healing in euglycemic rats, Journal of Surgical Research (2016) 206, 418-426). Impairment of proliferation of skin fibroblasts leads to delayed wound healing (Buranasin, P., High glucose-induced oxidative stress impairs proliferation and migration of human gingival fibroblasts, PLoS ONE (2018) 13), similar to the delay in wound healing observed in the skin of the elderly.

There is also evidence that an increase in glucose concentration leads to the terminal differentiation and change in morphology of these keratinocytes (Spravchikov, N. et al. Glucose effects on skin keratinocytes, Diabetes (2001) 50, 1627-1635). These terminally differentiated cells lose their ability to generate new cells and are arrested in a terminal state through the upregulation of cyclin-dependent kinase inhibitors and downregulation of positive mediators of the cell cycle.

Hyperglycemia, often accompanied with insulin deficiency or insulin resistance, also causes impaired autoregulation and increased permeability in microvessels.

Regarding cortisol, in the skin, elevated levels of glucocorticoids translate to lower permeability barrier homeostasis, less stratum corneum cohesion, decreased wound healing, and depressed innate immunity in the epidermis (Altemus, M., Stress-induced changes in skin barrier function in healthy women, Journal of Investigative dermatology (2001) 117, 309-317), all of which in turn could lead to intrinsic aging of the skin. Furthermore, sleep deprivation can cause physiological stress which induces excess glucocorticoid secretion and thus enhances skin aging. The excess glucocorticoids also inhibit the synthesis of lipids, causing lower production and secretion of lamellar bodies which can lead to further damage to the epidermal barrier.

Skin cells express both membrane bound and nuclear melatonin receptors which allow melatonin to play a role in multiple vital functions such as hair growth cycling, hair pigmentation, melanoma control, antioxidant activity, and suppression of ultraviolet-induced damage to skin cells (Slominski, A., On the role of melatonin in skin physiology and pathology, Endocrine (2005) 27, 137-148). Melatonin has been shown to decrease aging-related skin changes from oxidative stress and prevent extrinsic aging of the skin through protection against UV-induced skin aging. Dysregulation of melatonin ultimately has an adverse effect on the integrity of the skin and prevents melatonin's natural antiaging benefits on the skin. This leads to both increases in extrinsic aging of the skin from UV damage as well as intrinsic aging of the skin from oxidative stress.

Additionally, melatonin has antioxidant and scavenger properties (Reiter, R.J., Melatonin as an antioxidant: biochemical mechanisms and pathophysiological implications in humans, Acta Biochim Pol (2003) 50, 1129-1146).

Melatonin also reduces hyperglycemia damage on keratinocytes, reducing proinflammatory cytokines levels (Song, R., Melatonin promotes diabetic wound healing in vitro by regulating keratinocyte activity, Am J Transl Res (2016) 8, 4682-4693).

In recent years, the number of active ingredients to improve signs of skin aging and/or skin imperfections has increased considerably. Examples of such active ingredients are retinoids, vitamins or botanical extracts (Bradley E.J., Griffiths C.E.M., Sherratt M.J., Bell M. and Watson R.E.B. (2015), Over-the-counter anti-ageing topical agents and their ability to protect and repair photoaged skin. Maturitas, 80, 265-272).

In the case of retinoids, noteworthy is retinoic acid which is nowadays the 'gold-standard' for the induction of synthesis of several molecules of the ECM (for example, collagen, fibronectin or laminin; Varani J., Mitra R.S., Gibbs D., Phan S.H., Dixit V.M., Mitra R., Wang T., Siebert K.J., Nickoloff B.J., Voorhees J.J. (1990), All-Trans Retinoic Acid Stimulates Growth and Extracellular Matrix Production in Growth-Inhibited Cultured Human Skin Fibroblasts. The Journal of Investigative Dermatology, 94, 717-723). Hence, retinoic acid has been considered one of the most powerful compounds to treat the signs of aging, as it can significantly improve the clinical appearance of facial wrinkles by up-regulating the transcription and synthesis of proteins of the ECM such as collagen and fibronectin, and the inhibition of matrix metalloproteinases (hereinafter, MMP) (Varani J., Mitra R.S., Gibbs D., Phan S.H., Dixit V.M., Mitra R., Wang T., Siebert K.J., Nickoloff B.J., Voorhees J.J. (1990), All-Trans Retinoic Acid Stimulates Growth and Extracellular Matrix Production in Growth-Inhibited Cultured Human Skin Fibroblasts. The Journal of Investigative Dermatology, 94, 717-723). However, there are several drawbacks in the use of retinoic acid as, for example: retinoic acid has to be used cautiously as it can easily produce skin irritation and it is not recommended to combine retinoic acid and sun exposure. Another major concern when using retinoids is their instability, especially in the presence of oxygen and light (Sorg O., Antille C., Kaya G. and Saurat J-H. (2006), Retinoids in cosmeceuticals. Dermatologic Therapy, 19, 289-296).

Antioxidants are also used in order to reduce the concentration of free radicals in the skin and, therefore, counteract collagen degradation. An example of said antioxidants is ascorbic acid (also known as Vitamin C). Ascorbic acid, in addition to its antioxidant effect, induces the synthesis of proteins from the ECM (collagen I and III and elastin) while promoting epidermal differentiation and inhibiting Matrix metalloproteinase-1 (MMP1), among others. Unfortunately, ascorbic acid, is extremely unstable and undergoes oxidation especially at high temperatures, aerobic conditions, high pH and/or when exposed to light (Manela-Azulay M., Azulay V., Aguinaga F., Issa M.C. (2017), Vitamins and other Antioxidants. Daily Routine in Cosmetic Dermatology, 1-13).

In addition to chemically synthesized compounds, a wide range of botanical extracts and plant derived compounds are found in the market with multiple applications, such as, for example, grape extracts which comprise resveratrol (an antioxidant); green tea which comprises polyphenols; or soy which comprises isoflavones. However, the in vivo efficacy and composition of these ingredients is not sufficiently scientifically validated.

Hyaluronic acid, due to its viscoelastic properties and its capacity to retain water, has also been used in the cosmetic industry to keep skin hydrated, maintain elasticity and treat wrinkles by improving the roughness or even used as a dermal filler. However, currently, hyaluronic acid, is obtained from several sources, such as rooster combs or bacterial extracts and, consequently, these products can contain impurities and need to be characterized thoroughly (Kogan G., Soltés L., Stern R. and Gemeiner P. (2007), Hyaluronic acid: a natural biopolymer with a broad range of biomedical and industrial applications. Biotechnological Letters 29, 17-25).

On the other hand, peptides can also be incorporated in cosmetic formulas to improve the signs of skin aging. Bioactive peptides can imitate body's own molecules and influence processes such as collagen synthesis, with the advantage that they have much better tolerability and stability. In addition, a wide range of activities, chemistries and indications can be developed for them (Zhang L. and Falla T.J. (2009), Cosmeceuticals and peptides. Clinics in dermatology, 27, 485-494).

In addition, Japanese Patent application JP2011098900A discloses synthetic peptides which provide for the induction of differentiation in undifferentiated cells, mainly embryonic stem cells and induced pluripotent stem cells. The synthetic peptides comprise (A) an amino acid sequence of a membrane permeability peptide and (B) a signal peptide region of ephrins. Also disclosed in this document are methods for the differentiation of an undifferentiated cell and methods of producing a tissue.

European Patent application EP2740484A1, on its side, discloses a series of peptides which are useful for accelerating the DNA protection and reparation process stimulating the production or synthesis of proteins regulated by FOXO which intervene in said procedures. The peptides disclosed in this document have application both in medicine (for example, for the treatment of cancer) and in cosmetics (for example, for the treatment of aging or photoaging of the skin).

Moreover, United States Patent US5958752A refers to the discovery of the sequences of human proteins: trichohyalin and transglutaminase-3; and the sequence of the mouse transglutaminase-3. This document also refers to the combined use of trichohyalian and transglutaminase-3 to provide for the cross-linking of trichohyalin and, hence, a gel (proteinaceous). Also disclosed in D3 is the use of said combination to provide a method of facilitating the healing of a wound.

Despite the extensive variety of compounds and/or extracts in the field, there is still the need for improved active agents and compositions with novel mechanisms of action which allow the prevention, reduction and/or elimination of the signs of skin aging and/or skin imperfections, more precisely, there is the need to find new molecules (preferably, biomolecules) which target IL-33 and provide for the desired and required cosmetic and medical effects.

The inventors of the present invention, after extensive and exhaustive research, have surprisingly found peptides which are able to modulate the expression of IL-33 (downregulate) and which are, therefore, useful in cosmetics and in medicine. The peptides of the present invention are able to prevent, reduce and/or eliminate skin aging signs and/or skin imperfections, more preferably, facial skin aging, more preferably skin aging signs and/or skin imperfections in the eyes or the surroundings thereof, even more preferably, eyebags, eyelid imperfections, wrinkles in the surrounding of the eyes and dark circles (periorbital hyperchromia). In addition, the peptides of the present invention are also surprisingly able to prevent, reduce and/or eliminate the signs of skin aging and/or skin imperfections produced by sleep deprivation. As noted above, the peptides of the present invention due to their activities, are also useful in medicine.

In a first aspect, the present invention refers to peptides able to modulate the expression of IL-33 (downregulate). As noted above, the peptides of the present invention are useful in cosmetics and medicine, for the prevention, reduction and/or treatment of skin aging signs and/or skin imperfections.

In addition, in a second aspect, the present invention refers to a composition comprising at least one peptide of the present invention.

In a third aspect, the present invention refers to the use as a cosmetic of a peptide or a cosmetic composition of the present invention.

In a fourth aspect, the present invention refers to the cosmetic use of a peptide or a cosmetic composition of the present invention.

In a fifth aspect the present invention refers to a method for the cosmetic treatment of a subject in need thereof comprising the use of a peptide or a cosmetic composition of the present invention.

In a sixth aspect, the present invention refers to the peptides or pharmaceutical compositions of the present invention for use as a medicament.

The term "non-cyclic aliphatic group" and its plural, as used herein, have the common meaning given in the state of the art to said terms. Therefore, these terms refer to, for example and not restricted to, linear or branched alkyl, alkenyl and alkynyl groups.

The term "alkyl group" and its plural, as used herein, refer to a saturated, linear or branched group, which has between 1 and 24, preferably between 1 and 16, more preferably between 1 and 14, even more preferably between 1 and 12, and even more preferably still between 1, 2, 3, 4, 5 or 6 carbon atoms and which is bound to the rest of the molecule by a simple bond, including, for example and not restricted to, methyl, ethyl, isopropyl, n-propyl, i-propyl, isobutyl, tert-butyl, n-butyl, sec-butyl, n-pentyl, n-hexyl, heptyl, octyl, decyl, dodecyl, lauryl, hexadecyl, octadecyl, amyl, 2-ethylhexyl, 2-methylbutyl, 5-methylhexyl and similar. The alkyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "alkenyl group" and its plural, as used herein, refer to a linear or branched group which has between 2 and 24, preferably between 2 and 16, more preferably between 2 and 14, even more preferably between 2 and 12, even more preferably still 2, 3, 4, 5 or 6 carbon atoms, with one or more carbon-carbon double bonds, preferably with 1, 2 or 3 carbon-carbon double bonds, conjugated or unconjugated, which is bound to the rest of the molecule through a single bond, including, for example and not restricted to, the vinyl, oleyl, linoleyl and similar groups. The alkenyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "alkynyl group" and its plural, as used herein, refer to a linear or branched group which has between 2 and 24, preferably between 2 and 16, more preferably between 2 and 14, even more preferably between 2 and 12, even more preferably still 2, 3, 4, 5 or 6 carbon atoms, with one or more carbon-carbon triple bonds, preferably with 1, 2 or 3 carbon-carbon triple bonds, conjugated or unconjugated, which is bound to the rest of the molecule through a single bond, including, for example and not restricted to, the ethinyl group, 1-propinyl, 2-propinyl, 1-butinyl, 2-butinyl, 3-butinyl, pentinyl, such as 1-pentinyl and similar groups. The alkynyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "alicyclic group" and its plural, as used herein, have the common meaning given in the state of the art to said terms. Hence, these terms are used to refer to, for example and not restricted to, cycloalkyl or cycloalkenyl or cycloalkynyl groups.

The term "cycloalkyl" and its plural, as used herein, refer to a saturated mono- or polycyclic aliphatic group which has between 3 and 24, preferably between 3 and 16, more preferably between 3 and 14, even more preferably between 3 and 12, even more preferably still 3, 4, 5 or 6 carbon atoms and which is bound to the rest of the molecule through a single bond, including, for example and not limited to, cyclopropyl, cyclobutyl, cyclopentyl, cyclohexyl, cycloheptyl, methyl cyclohexyl, dimethyl cyclohexyl, octahydroindene, decahydronaphthalene, dodecahydro-phenalene, adamantyl and similar, and that can optionally be substituted by one or more groups, such as, alkyl, halo, hydroxy, alkoxy, carboxy, carbonyl, cyano, acyl, alkoxy-carbonyl, amino, nitro, mercapto and alkoxythio.

The term "cycloalkenyl" and its plural, as used herein, refer to a non-aromatic mono- or polycyclic aliphatic group which has between 5 and 24, preferably between 5 and 16, more preferably between 5 and 14, even more preferably between 5 and 12, even more preferably still 5 or 6 carbon atoms, with one or more carbon-carbon double bonds, preferably with 1, 2 or 3 carbon-carbon double bonds, conjugated or unconjugated, which is bound to the rest of the molecule through a single bond, including, for example and not restricted to, the cyclopent-1-en-1-yl group and similar groups, and that can optionally be substituted by one or more groups, such as, alkyl, halo, hydroxy, alkoxy, carboxy, carbonyl, cyano, acyl, alkoxy-carbonyl, amino, nitro, mercapto and alkoxythio.

The term "cycloalkynyl" and its plural, as used herein, refer to a non-aromatic mono- or polycyclic aliphatic group which has between 8 and 24, preferably between 8 and 16, more preferably between 8 and 14, even more preferably between 8 and 12, even more preferably still 8 or 9 carbon atoms, with one or more carbon-carbon triple bonds, preferably with 1, 2 or 3 carbon-carbon triple bonds, conjugated or unconjugated, which is bound to the rest of the molecule through a single bond, including, for example and not restricted to, the cyclooct-2-yn-1-yl group and similar, and that can optionally be substituted by one or more groups, such as, alkyl, halo, hydroxy, alkoxy, carboxy, carbonyl, cyano, acyl, alkoxy-carbonyl, amino, nitro, mercapto and alkoxythio.

The term "aryl group" and its plural, as used herein, refer to an aromatic group which has between 6 and 30, preferably between 6 and 18, more preferably between 6 and 10, even more preferably 6 or 10 carbon atoms, which comprises 1, 2, 3 or 4 aromatic rings, bound by a carbon-carbon bond or fused, and which is bound to the rest of the molecule through a single bond, including, for example and not restricted to, phenyl, naphthyl, diphenyl, indenyl, phenanthryl or anthranyl among others. The aryl group can be optionally substituted by one or more substituents, such as, halo, hydroxy, alkoxy, carboxy, carbonyl, cyano, acyl, alkoxy-carbonyl, amino, nitro, mercapto and alkoxythio.

The term "aralkyl group" and its plural, as used herein, refer to an alkyl group substituted by an aromatic group, with between 7 and 24 carbon atoms and including, for example and not restricted to, -(CH₂)1-6-phenyl, -(CH₂)1-6-(1-naphtyl), -(CH₂)1-6-(2-naphtyl), - (CH₂)1-6-CH(phenyl)₂ and similar. The aralkyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "heterocyclic group" and its plural, as used herein, refer to a 3-10 member heterocycyl or hydrocarbon ring, in which one or more of the ring atoms, preferably 1, 2 or 3 of the ring atoms, is a different element to carbon, such as nitrogen, oxygen or sulfur and may be saturated or unsaturated. For the purposes of this invention, the heterocyclyl can be a cyclic, monocyclic, bicyclic or tricyclic system which may include fused ring systems; and the nitrogen, carbon or sulfur atoms can be optionally oxidized in the heterocyclyl radical; the nitrogen atom can optionally be quaternized; and the heterocyclyl radical may be partially or completely saturated or may be aromatic. With increasing preference, the term heterocyclic relates to a 5 or 6-member ring. The heterocyclic groups can be optionally substituted by one or more substituents, such as, halo, hydroxy, alkoxy, carboxy, carbonyl, cyano, acyl, alkoxy-carbonyl, amino, nitro, mercapto and alkoxythio.

The term "heteroarylalkyl group" and its plural, as used herein, refer to an alkyl group substituted with a substituted or unsubstituted aromatic heterocyclyl group, the alkyl group having from 1 to 6 carbon atoms and the aromatic heterocyclyl group between 2 and 24 carbon atoms and from 1 to 3 atoms other than carbon and including, for example and not restricted to, -(CH₂)1-6-imidazolyl, -(CH₂)1-6-triazolyl, -(CH₂)1-6-thienyl, - (CH₂)1-6-furyl, -(CH₂)1-6-pyrrolidinyl and similar. The heteroarylalkyl groups can be optionally substituted by one or more substituents, such as, halo, hydroxy, alkoxy, carboxy, carbonyl, cyano, acyl, alkoxy-carbonyl, amino, nitro, mercapto and alkoxythio.

The terms "halo" or "halogen", as used in the present document, refer to fluorine, chlorine, bromine or iodine, and its anions are referred to as halides.

As used in the present document, the term "salt" and its plurals refer to any type of salt from among those known in the state of the art, for example, halide salts, hydroxy acid salts (such as oxyacid salts, acid salts, basic salts and double salts), hydroxo salts, mixed salts, oxy salts or other hydrated salts. This term comprises both cosmetically and/or pharmaceutically acceptable salts; and cosmetically and/or pharmaceutically unacceptable salts, since the latter may be useful in the preparation of cosmetically and/or pharmaceutically acceptable salts.

As used in the present document, the term "isomer" and its plural refer to optical isomers, enantiomers, stereoisomers or diastereoisomers. The individual enantiomers or diastereoisomers, as well as their mixtures, may be separated by conventional techniques known in the state of the art.

As used herein, the term "solvate" and its plural refer to any solvate known in the state of the art, such as polar, apolar or amphiphilic solvates, and include any cosmetically acceptable solvate which, when administered or applied to the interested subject (directly or indirectly) provides the compound of interest (the peptide or peptides of the present invention). Preferably, the solvate is a hydrate, a solvate with an alcohol such as methanol, ethanol, propanol or isopropanol, a solvate with an ester such as ethyl acetate, a solvate with an ether such as methyl ether, ethyl ether or THF (tetrahydrofuran) or a solvate with DMF (dimethylformamide), and more preferably a hydrate or a solvate with an alcohol such as ethanol.

In addition, as used herein, the term "amino acid" and its plural include the amino acids codified by the genetic code as well as uncodified amino acids, whether they are natural or not and whether they are D- and L-amino acids. Examples of uncodified amino acids are, without restriction, citrulline, ornithine, sarcosine, desmosine, norvaline, 4-aminobutyric acid, 2-aminobutyric acid, 2-aminoisobutyric acid, 6-aminohexanoic acid, 1-naphthylalanine, 2-naphthylalanine, 2-aminobenzoic acid, 4 aminobenzoic acid, 4-chlorophenylalanine, 2,3-diaminopropionic acid, 2,4 diaminobutyric acid, cycloserine, carnitine, cysteine, penicillamine, pyroglutamic acid, thienylalanine, hydroxyproline, allo-isoleucine, allo-threonine, isonipecotic acid, isoserine, phenylglycine, statin, β-alanine, norleucine, N-methylamino acids, α-amino acids and β-amino acids, among others, as well as their derivatives. Nevertheless, further unnatural amino acids are known in the state of the art (see, for example, *"*Unusual amino acids in peptide synthesis" by D. C. Roberts and F. Vellaccio, The Peptides, Vol. 5 (1983), Chapter VI, Gross E. and Meienhofer J., Eds., Academic Press, New York, USA).

The "percentage of identity" regarding peptides, polypeptides and proteins, as used herein, has the meaning commonly attributed in the state of the art and, hence, relates to the percentage of amino acids which are identical between two amino acid sequences which are compared after an optimal alignment of these sequences, where said percentage is merely statistical and the differences between the two amino acid sequences are randomly distributed throughout the sequence. "Optimal alignment" is understood as that alignment of amino acid sequences giving rise to a greater percentage of identity. The percentage of identity is calculated by determining the number of identical positions in which an amino acid is identical in the two compared sequences, dividing the number of identical positions by the number of compared positions and multiplying the result obtained by 100 to obtain the percentage of identity between the two sequences. The sequence comparisons between two amino acid sequences can be carried out manually or by means of computer programs known in the state of the art, such as the BLAST (Basic Local Alignment Search Tool) algorithm.

As used herein, "dark circles" and "periorbital hyperchromia" are used interchangeably and acquire the meaning they commonly have in the state of the art. Briefly, dark circles or periorbital hyperchromia are normally characterized by a periorbital melanin deposition, a bluish colour and visibility of the dermal capillary network around the eyes and a loss of subcutaneous fat providing for a thinning of the skin and cheek descent.

As used herein, IL33 refers to the gene that encodes the protein Interleukin-33.

As used herein, IL-33 refers to the protein Interleukin-33.

As stated above, in a first aspect, he peptide of the present invention has a sequence in accordance with formula (I):

R₁-AA₁-AA₂-AA₃-AA₄-AA₅-AA₆-R₂

wherein:
the 6 amino acids are 3 arginines, 1 glutamine, 1 methionine and 1 glutamic acid
and wherein:
   - R₁ is selected from H, substituted or unsubstituted non-cyclic aliphatic, substituted or unsubstituted alicyclyl, substituted or unsubstituted heterocyclyl, substituted or unsubstituted heteroarylalkyl, substituted or unsubstituted aryl, substituted or unsubstituted aralkyl and R₅-CO-, wherein R₅ is selected from the group formed by substituted or unsubstituted C₁-C₂₄ alkyl radical, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, substituted or unsubstituted heterocyclyl ring of 3 to 10 members, and substituted or unsubstituted heteroarylalkyl of 2 to 24 carbon atoms and 1 to 3 atoms other than carbon and an alkyl chain of 1 to 6 carbon atoms; and
   - R₂ is selected from H, -NR₃R₄- , -OR₃ and -SR₃, wherein R₃ and R₄ are independently selected from H, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl.

As noted above, the peptide of the present invention modulates the expression of gene IL33 and/or of protein Interleukin-33, more preferably, downregulate the expression of gene IL33 and/or of protein Interleukin-33.

Also included within the present invention are isomers, salts, solvates and mixtures thereof of the peptides of the present invention; more preferably, cosmetically and/or pharmaceutically acceptable isomers, salts, solvates and mixtures thereof of the peptides of the present invention.

It is contemplated that the amino acids used or present in the peptides of the present invention are L-amino acids, D-amino acids or combinations thereof. In a preferred embodiment, the amino acids used or present in the peptides of the present invention are L-amino acids.

Preferably, the isomers mentioned above are stereoisomers. It is contemplated that said stereoisomers are enantiomers or diastereoisomers. Hence, in a preferred embodiment of the present invention, the peptide is a racemic mixture, a diastereomeric mixture, a pure enantiomer or a pure diastereoisomer.

R₁ is preferably selected from H or R₅-CO-, wherein R₅ is selected from the group formed by substituted or unsubstituted C₁-C₂₄ alkyl radical, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, substituted or unsubstituted heterocyclyl ring of 3 to 10 members, and substituted or unsubstituted heteroarylalkyl of 2 to 24 carbon atoms and 1 to 3 atoms other than carbon and an alkyl chain of 1 to 6 carbon atoms. Even more preferably, R₁ is selected from H, acetyl (hereinafter, Ac), tert-butanoyl, hexanoyl, 2-methylhexanoyl, cyclohexanecarboxyl, octanoyl, decanoyl, lauroyl myristoyl, palmitoyl (hereinafter, Pal), stearoyl, oleoyl and linoleoyl. Even more preferably, R₁ is Ac.

Preferably, R₂ is NH₂.

Therefore, more preferably, R₁ is Ac and R₂ is NH₂.

Preferably, the peptide the peptide of the present invention comprises Arg-Arg, Arg-Glu, Met-Arg or combinations thereof within its sequence. In a most preferred embodiment, the peptide of the present invention comprises Arg-Arg, Arg-Glu and Met-Arg within its sequence.

In one of the most preferred embodiments, the sequence of the peptide of the present invention is:
R₁-Arg-Arg-Gln-Met-Arg-Glu-R₂ (R₁-SEQ ID NO:1-R₂);
R₁-Met-Arg-Arg-Glu-Gln-Arg-R₂ (R₁-SEQ ID NO:2-R₂); or
R₁-Arg-Glu-Gln-Met-Arg-Arg-R₂ (R₁-SEQ ID NO:3-R₂).

As stated above, R₁ is preferably selected from H or R₅-CO-, wherein R₅ is selected from the group formed by substituted or unsubstituted C₁-C₂₄ alkyl radical, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, substituted or unsubstituted heterocyclyl ring of 3 to 10 members, and substituted or unsubstituted heteroarylalkyl of 2 to 24 carbon atoms and 1 to 3 atoms other than carbon and an alkyl chain of 1 to 6 carbon atoms. Even more preferably, R₁ is selected from H, acetyl (hereinafter, Ac), tert-butanoyl, hexanoyl, 2-methylhexanoyl, cyclohexanecarboxyl, octanoyl, decanoyl, lauroyl myristoyl, palmitoyl (hereinafter, Pal), stearoyl, oleoyl and linoleoyl, even more preferably, R₁ is Ac.

Also as stated above, R₂ is, preferably, NH₂.

Therefore, in the most preferred embodiment, the sequence of the peptide of the present invention is:
Ac-Arg-Arg-Gln-Met-Arg-Glu-NH₂ (Ac-SEQ ID NO: 1-NH₂);
Ac-Met-Arg-Arg-Glu-Gln-Arg-NH₂ (Ac-SEQ ID NO: 2-NH₂); or
Ac-Arg-Glu-Gln-Met-Arg-Arg-NH₂ (Ac-SEQ ID NO: 3-NH₂).

As can be directly derivable from the examples included below, the peptides of the present invention are able to downregulate the expression of gene IL33 and of the protein IL-33. Also, the peptides of the present invention provide for a favorable regulation of extracellular matrix related genes, inhibition of tyrosinase activity and protection against protein glycation and lipid peroxidation. Therefore, the peptides of the present invention are useful in cosmetics and medicine for the treatment of signs of skin aging and/or skin imperfections, more precisely:
- In cosmetics, given the activities mentioned above and demonstrated in the examples included below, the peptides of the present invention are useful, at least, for the treatment of eyebags, eyelid imperfections, wrinkles (preferably, wrinkles in the surrounding of the eyes) and hyperchromia (preferably, periorbital hyperchromia. Also, the peptides of the present invention are able to prevent, reduce and/or revert the effects in the skin of sleep deprivation, this is, they are able to treat the signs of skin aging and/or skin imperfections associated with sleep deprivation.
- In medicine, given the activities mentioned above and demonstrated in the examples included below, the peptides of the present invention are useful for the treatment of medical conditions related with inflammation, angiogenesis and/or hyperpigmentation, diseases related with lipid peroxidation, diseases related with protein glycation or combinations thereof. Most preferably, the peptides of the present invention are useful, at least, for the treatment of eyelid imperfections, more preferably, dermatochalasis, ptosis (for example, aponeurotic ptosis and aponeurotic blepharoptosis), ectropion and entropion.

Hence, the peptides of the present invention solve the needs and technical problems present in the state of the art and mentioned above.

In addition, also included within the scope of the present invention are peptides with a 70% percentage identity, preferably 80%, more preferably 90%, more preferably 95%, even more preferably 99% percentage identity with any of the specific sequences mentioned above (this is, R₁-Arg-Arg-Gln-Met-Arg-Glu-R₂; R₁-Met-Arg-Arg-Glu-Gln-Arg-R₂; or R₁-Arg-Glu-Gln-Met-Arg-Arg-R₂, preferably, Ac-Arg-Arg-Gln-Met-Arg-Glu-NH₂; Ac-Met-Arg-Arg-Glu-Gln-Arg-NH₂; or Ac-Arg-Glu-Gln-Met-Arg-Arg-NH₂) and which still show the activities described herein for the peptides of the present invention.

The peptides of the present invention may be synthesized and produced by any means known in the state of the art. For example, they may be synthesized and produced by chemical synthesis (preferably, by means of solid phase peptide synthesis), expressing said peptides in cell cultures or by means of transgenic production of the peptide in plants or animals. In addition, the peptides of the present invention may be purified by any means known in the state of the art.

In a preferred embodiment, the peptide of the present invention is suited or adapted to be applied by means of iontophoresis, more preferably, in the face of a subject (preferably, a human), more preferably in and/or around the eyes.

In a further preferred embodiment, the peptide of the present invention is suited or adapted to be applied subcutaneously, more preferably, in the face of a subject (preferably, a human), more preferably in and/or around the eyes.

In another preferred embodiment, the peptide of the present invention is suited or adapted to be applied topically (preferably, in the form of a cream), more preferably, in the face of a subject (preferably, a human), more preferably in and/or around the eyes.

In a second aspect, the present invention refers to a composition comprising at least one peptide in accordance with the present invention.

It is contemplated that the composition of the present invention comprises one type of peptide of the present invention or a combination or mixture of different peptides of the present invention, preferably, one type of peptide of the present invention.

In a preferred embodiment, the composition of the present invention is a cosmetic composition. Hence, in this embodiment, the cosmetic composition of the present invention comprises a cosmetically effective amount of the at least one peptide of the present invention, more preferably the cosmetic composition of the present invention comprises from 0.0001% (weight/volume in g/100mL, hereinafter, w/v) to 0.05% (w/v) of at least one peptide of the present invention. In a most preferred embodiment, the cosmetic composition of the present invention comprises from 0.0001 % (w/v) to 0.001% (w/v) of at least one peptide of the present invention, more preferably, 0.0005% (w/v). In another most preferred embodiment, the cosmetic composition of the present invention comprises from 0.05% (w/v) to 0.001% (w/v) of at least one peptide of the present invention.

It is contemplated that the cosmetic composition of the present invention also comprises at least one additional cosmetic ingredient. Said additional cosmetic ingredient can be at least one excipient and/or at least one additional cosmetic active ingredient.

The additional cosmetic ingredients comprise those usually used in the state of the art as, for example, adjuvants such as stabilizer, solubilizer, vitamin, colorant and perfumery; carriers; and/or other cosmetic active ingredients.

Said additional cosmetic ingredients, must be physically and chemically compatible with the rest of the components of the composition and, especially, with the peptides of the present invention comprised in the composition of the present invention. Likewise, the nature of said additional cosmetic ingredients must not unacceptably alter the benefits of the peptides and compositions of the present invention. Said additional cosmetic ingredients may be of a synthetic or natural origin, such as, for example, plant extracts, or they can be derived from a biofermentation process (see, for example, CTFA Cosmetic Ingredient Handbook, Eleventh Edition (2006)).

It is contemplated that the additional cosmetic ingredients mentioned above comprise those ingredients commonly used in compositions for caring for; cleaning skin and/or hair; and/or deodorants and/or creams to prevent hyperhidrosis; such as, for example, agents inhibiting melanin synthesis, whitening or depigmenting agents, anti-aging agents, agents inhibiting NO-synthase, antioxidants, anti-atmospheric pollution and/or free radical trapping agents, anti-glycation agents, emulsifying agents, emollients, organic solvents, liquid propellants, skin conditioners such as for example wetting agents, moisture retaining substances, alpha hydroxy acids, moisturizers, vitamins, pigments or colorants, dyes, gelling polymers, thickeners, surfactants, softeners, other anti-wrinkle agents, agents capable of reducing or eliminating bags under the eyes, exfoliating agents, antimicrobial agents, antifungal agents, bactericides, agents stimulating dermal or epidermal macromolecule synthesis and/or capable of preventing or inhibiting their degradation, such as for example agents stimulating collagens synthesis, agents stimulating elastin synthesis, agents stimulating laminin synthesis, agents inhibiting collagen degradation, agents inhibiting elastin degradation, agents stimulating fibroblast proliferation, agents stimulating keratinocyte proliferation, agents stimulating keratinocyte differentiation, agents stimulating lipid synthesis and synthesis of components of the *stratum corneum* (ceramides, fatty acids, etc.), dermorelaxing agents, agents stimulating glycosaminoglycan synthesis, DNA repairing agents, DNA protecting agents, agents stimulating proteosome activity, anti-pruritus agents, agents for treating sensitive skin, reaffirming agents, astringent agents, sebum production regulating agents, agents stimulating lipolysis, anti-cellulite agents, calming agents, antiinflammatory agents, agents acting on capillary circulation and/or microcirculation, agents acting on cell mitochondria, agents intended to improve the dermo-epidermal junction, preservatives, perfumes, chelating agents, plant extracts, essential oils, marine extracts, agents derived from a biofermentation process, mineral salts, cell extracts and/or solar filters (organic or mineral photoprotective agents active against ultraviolet A and B rays) among others.

In an embodiment, at least one of the additional cosmetic ingredients is a cosmetic active principle or substance which may exert the same, similar, complementary or different cosmetic activities as those disclosed above for the peptides of the present invention. It is contemplated that the composition of the present invention comprises other anti-wrinkling or anti-aging agents, for example, collagen, elastin, growth factors, hyaluronic acid boosters, barrier function agents, illuminating agents, agents stimulating the expression and/or synthesis of collagen I, III, IV and/or VI and laminin; agents stimulating the synthesis of glycosaminoglycans or hyaluronic acid; agents stimulating the expression and/or synthesis of elastin and other elastic fibres-related proteins; agents inhibiting collagen and/or elastic fibres degradation; agents stimulating the expression and/or synthesis of mitochondria-related proteins (for example, sirtuins and aconitase); agents stimulating the expression and/or synthesis of focal adhesion proteins; agents stimulating keratinocytes and/or fibroblasts proliferation and/or differentiation; antioxidants; anti-atmospheric pollution and/or free radical trapping agents; anti-glycation agents; detoxifying agents; agents decreasing chronological aging, environmental aging and inflammation aging; and agents decreasing melanin production and/or inhibiting tyrosinase and/or agents stimulating lipid synthesis and synthesis of components of the epidermis (keratins) and more specifically the stratum corneum (keratins, ceramides, filaggrin, loricrin and SPRR1B). More preferably, the at least one of the additional cosmetic ingredients is Argireline^{®} (Acetyl Hexapeptide-8), Leuphasyl^{®} (Pentapeptide-3), Inyline^{®} (Acetyl Hexapeptide-30), Syn-Ake^{®} (Tripeptide-3) or combinations thereof.

In addition, the cosmetic composition of the present invention (or the peptide of the present invention) can be formulated in any form usually used in the state of the art as, for example, solution, suspension, emulsion, paste, gel, cream, powder, spray, lotion, oil, liniment, serum, mousse, ointment, bar or pencil including "leave on" and "rinse-off" formulations. The cosmetic composition of the present invention can also be incorporated by means of techniques known in the state of the art to different types of solid accessories such as towelettes, hydrogels, adhesive (or non-adhesive) patches or face masks, or it could be incorporated to different make-up line products such as concealers, make-up foundations, lotions or make-up removal lotions, among others.

It is also contemplated that the cosmetic composition of the present invention or a peptide of the present invention, both as disclosed herein, can also be incorporated in cosmetic sustained release systems and/or carriers such as liposomes, milliparticles, microparticles and nanoparticles, as well as in sponges, vesicles, micelles, millispheres, microspheres, nanospheres, lipospheres, millicapsules, microcapsules and nanocapsules, as well as in microemulsions and nanoemulsions, for the purpose of obtaining greater penetration of the active ingredient.

In a preferred embodiment, the cosmetic composition of the present invention is suited or adapted to be applied by means of iontophoresis, more preferably, in the face of a subject (preferably, a human), more preferably in and/or around the eyes.

In a further preferred embodiment, the cosmetic composition of the present invention is suited or adapted to be applied subcutaneously, more preferably, in the face of a subject (preferably, a human), more preferably in and/or around the eyes.

In another preferred embodiment, the cosmetic composition of the present invention is suited or adapted to be applied topically (preferably, in the form of a cream), more preferably, in the face of a subject (preferably, a human), more preferably in and/or around the eyes.

In another preferred embodiment, the composition of the present invention is a pharmaceutical composition. Hence, in this embodiment, the pharmaceutical composition of the present invention comprises a pharmaceutically effective amount of the at least one peptide of the present invention, more preferably the pharmaceutical composition of the present invention comprises from 0.0001 % (w/v) to 0.05% (w/v) of at least one peptide of the present invention. In a most preferred embodiment, the pharmaceutical composition of the present invention comprises from 0.0001% (w/v) to 0.001% (w/v) of at least one peptide of the present invention, more preferably, 0.0005% (w/v). In another most preferred embodiment, the pharmaceutical composition of the present invention comprises from 0.05% (w/v) to 0.001 % (w/v) of at least one peptide of the present.

The pharmaceutical composition can be in any form known in the state of the art which is suited for the chosen route. It is contemplated that the pharmaceutical composition of the present invention is suited or adapted to be administered by any means and any route known in the state of the art (for example, subcutaneously, intramuscularly, intravenously, topically or orally; in the latter case, for example, in the form of a saline solution and/or of a biodegradable material, such as polymers of polylactic acid (PLA), polyglycolic acid (PGA) polylactic acid-glycolic acid copolymers, polycaprolactones and/or cholesterol). More preferably, the pharmaceutical composition of the present invention is suited or adapted to be administered topically, even more preferably in the form of a cream.

It is contemplated that the pharmaceutical composition of the present invention also comprises at least one additional pharmaceutical ingredient. Said additional pharmaceutical ingredient can be at least one excipient and/or at least one additional pharmaceutical active ingredient.

As can be directly derivable from the examples included below, the peptides of the present invention are able to downregulate the expression of gene IL33 and of the protein IL-33. Also, the peptides of the present invention, provide for a favorable regulation of extracellular matrix related genes, inhibition of tyrosinase activity and protection against protein glycation and lipid peroxidation. Therefore, the compositions of the present invention are useful in cosmetics and medicine for the treatment of signs of skin aging and/or skin imperfections, more precisely:
- In cosmetics, given the activities mentioned above and demonstrated in the examples included below, the peptides of the present invention are useful, at least, for the treatment of eyebags, eyelid imperfections, wrinkles (preferably, wrinkles in the surrounding of the eyes) and hyperchromia (preferably, periorbital hyperchromia). Also, the peptides of the present invention are able to prevent, reduce and/or revert the effects in the skin of sleep deprivation, this is, they are able to treat the signs of skin aging and/or skin imperfections associated with sleep deprivation.
- In medicine, given the activities mentioned above and demonstrated in the examples included below, the peptides of the present invention are useful for the treatment of medical conditions related with inflammation, angiogenesis and/or hyperpigmentation, diseases related with lipid peroxidation, diseases related with protein glycation or combinations thereof. Most preferably, the peptides of the present invention are useful, at least for the treatment of eyelid imperfections, more preferably, dermatochalasis, ptosis (for example, aponeurotic ptosis and aponeurotic blepharoptosis), ectropion and entropion.

Hence, the composition of the present invention solves the needs and technical problems present in the state of the art and mentioned above.

As already stated above, in a third aspect the present invention refers to the use as a cosmetic of a peptide or of a cosmetic composition in accordance with the present invention.

The peptide and the cosmetic composition in accordance with the present invention are as explained above.

Preferably, the use as a cosmetic is for the treatment of cosmetic conditions related with inflammation, angiogenesis, extracellular matrix damage, oxidative stress, alterations in dermal cell junctions, apoptosis and/or hyperpigmentation, preferably cosmetic conditions related with cosmetic inflammation, cosmetic angiogenesis, cosmetic extracellular matrix damage, cosmetic oxidative stress, cosmetic alterations in dermal cell junctions, cosmetic apoptosis and/or cosmetic hyperpigmentation.

More preferably, the use as a cosmetic is for the treatment of skin aging signs and/or skin imperfections, more preferably, for the prevention, reduction and/or elimination of skin aging signs and/or skin imperfections.

As it is evident, the signs of skin aging mentioned above are cosmetic signs of skin aging and/or cosmetic skin imperfections.

Skin aging is due to chronological and/or environmental aging.

More preferably, said skin aging signs and/or skin imperfections are produced by a dysregulation of IL-33. As noted above, the peptides and cosmetic compositions of the present invention downregulate IL-33 (they downregulate the expression of IL33 gene and/or the expression of Interleukin 33).

In a most preferred embodiment, said skin aging signs and/or skin imperfections are eyebags, eyelid imperfections, wrinkles (preferably, wrinkles in the surrounding of the eyes), hyperchromia (preferably, periorbital hyperchromia) or combinations thereof.

Eyelid imperfections is preferably, sagging eyelid, more preferably, cosmetic dermatochalasis, cosmetic ptosis, cosmetic ectropion and cosmetic entropion, more preferably, cosmetic dermatochalasis, cosmetic aponeurotic ptosis, cosmetic aponeurotic blepharoptosis, cosmetic involutional ectropion and cosmetic involutional entropion.

Therefore, preferably, said skin aging signs and/or skin imperfections are eyebags, wrinkles in the surrounding of the eyes, periorbital hyperchromia, sagging eyelid or combinations thereof, more preferably, eyebags, wrinkles in the surrounding of the eyes, periorbital hyperchromia, cosmetic dermatochalasis, cosmetic aponeurotic ptosis, cosmetic aponeurotic blepharoptosis, cosmetic involutional ectropion and cosmetic involutional entropion or combinations thereof.

In the most preferred embodiment, said skin aging signs and/or skin imperfections are eyebags, wrinkles in the surrounding of the eyes, periorbital hyperchromia or combinations thereof.

In another most preferred embodiment, the use as a cosmetic is for the treatment of the effects in the skin of sleep deprivation, more preferably, to prevent, reduce and/or eliminate the signs of skin aging and/or skin imperfections associated with sleep deprivation.

The signs of skin aging and/or skin imperfections associated with sleep deprivation are preferably sagging eyelid (as explained above), periorbital hyperchromia, signs associated with impaired barrier function or combinations thereof.

In a preferred embodiment the signs of skin aging and/or skin imperfections associated with sleep deprivation are periorbital hyperchromia.

In another preferred embodiment the signs of skin aging and/or skin imperfections associated with sleep deprivation are signs associated with impaired barrier function, more preferably altered skin permeability, skin inflammation, lack of cellular cohesion in the skin or combinations thereof, even more preferably eyebags, wrinkles (preferably, wrinkles in the surrounding of the eyes) or combinations thereof.

Also, in a preferred embodiment, the use as a cosmetic is in a subject. Preferably, the subject is a mammal, even more preferably, a human.

It is contemplated that the peptide or the cosmetic composition of the present invention is applied by means of iontophoresis, more preferably in the face of the subject (preferably, a human), even more preferably in and/or around the eyes.

It is also contemplated that the peptide or the cosmetic composition of the present invention is applied subcutaneously, more preferably, in the face of a subject (preferably, a human), even more preferably in and/or around the eyes.

In the most preferred embodiment, the peptide or the cosmetic composition of the present invention is applied topically (preferably, in the form of a cream), more preferably, in the face of a subject (preferably, a human), even more preferably in and/or around the eyes.

In addition, in the use as a cosmetic of the present invention, the peptide or the cosmetic composition of the present invention are used in a cosmetically effective amount. More preferably, the peptide of the present invention is used at a concentration of 0.0001% (w/v) to 0.05% (w/v). In a most preferred embodiment, the peptide of the present invention is used at a concentration of 0.0001% (w/v) to 0.001 % (w/v), more preferably, 0.0005% (w/v). In another most preferred embodiment, the peptide of the present invention is used at a concentration of 0.05% (w/v) to 0.001% (w/v).

In a fourth aspect, the present invention refers to the cosmetic use of a peptide or a cosmetic composition in accordance with the present invention.

The peptide and the cosmetic composition in accordance with the present invention are as explained above.

Preferably, the cosmetic use is for the treatment of cosmetic conditions related with inflammation, angiogenesis, extracellular matrix damage, oxidative stress, alterations in dermal cell junctions, apoptosis and/or hyperpigmentation, preferably cosmetic conditions related with cosmetic inflammation, cosmetic angiogenesis, cosmetic extracellular matrix damage, cosmetic oxidative stress, cosmetic alterations in dermal cell junctions, cosmetic apoptosis and/or cosmetic hyperpigmentation.

More preferably, the cosmetic use is for the treatment of skin aging signs and/or skin imperfections, more preferably, for the prevention, reduction and/or elimination of skin aging signs and/or skin imperfections.

As it is evident, the signs of skin aging mentioned above are cosmetic signs of skin aging and/or cosmetic skin imperfections.

Skin aging is due to chronological and/or environmental aging.

More preferably, said skin aging signs and/or skin imperfections are produced by a dysregulation of IL-33. As noted above, the peptides and cosmetic compositions of the present invention downregulate IL-33 (they downregulate the expression of IL33 gene and/or the expression of Interleukin 33).

In a most preferred embodiment, said skin aging signs and/or skin imperfections are eyebags, eyelid imperfections, wrinkles (preferably, wrinkles in the surrounding of the eyes), hyperchromia (preferably, periorbital hyperchromia) or combinations thereof.

Eyelid imperfections is preferably, sagging eyelid, more preferably, cosmetic dermatochalasis, cosmetic ptosis, cosmetic ectropion and cosmetic entropion, more preferably, cosmetic dermatochalasis, cosmetic aponeurotic ptosis, cosmetic aponeurotic blepharoptosis, cosmetic involutional ectropion and cosmetic involutional entropion.

Therefore, preferably, said skin aging signs and/or skin imperfections are eyebags, wrinkles in the surrounding of the eyes, periorbital hyperchromia, sagging eyelid or combinations thereof, more preferably, eyebags, wrinkles in the surrounding of the eyes, periorbital hyperchromia, cosmetic dermatochalasis, cosmetic aponeurotic ptosis, cosmetic aponeurotic blepharoptosis, cosmetic involutional ectropion and cosmetic involutional entropion or combinations thereof.

In the most preferred embodiment, said skin aging signs and/or skin imperfections are eyebags, wrinkles in the surrounding of the eyes, periorbital hyperchromia or combinations thereof.

In another most preferred embodiment, the cosmetic use is for the treatment of the effects in the skin of sleep deprivation, more preferably, to prevent, reduce and/or eliminate the signs of skin aging and/or skin imperfections associated with sleep deprivation.

The signs of skin aging and/or skin imperfections associated with sleep deprivation are preferably sagging eyelid (as explained above), periorbital hyperchromia, signs associated with impaired barrier function or combinations thereof.

In a preferred embodiment the signs of skin aging and/or skin imperfections associated with sleep deprivation are periorbital hyperchromia.

In another preferred embodiment the signs of skin aging and/or skin imperfections associated with sleep deprivation are signs associated with impaired barrier function, more preferably altered skin permeability, skin inflammation, lack of cellular cohesion in the skin or combinations thereof, even more preferably eyebags, wrinkles (preferably, wrinkles in the surrounding of the eyes) or combinations thereof.

Also, in a preferred embodiment, the cosmetic use of the present invention is in a subject. More preferably, the subject is a mammal, even more preferably, a human.

It is contemplated that the peptide or the cosmetic composition of the present invention is applied by means of iontophoresis, more preferably in the face of the subject (preferably, a human), even more preferably in and/or around the eyes.

It is also contemplated that the peptide or the cosmetic composition of the present invention is applied subcutaneously, more preferably, in the face of a subject (preferably, a human), even more preferably in and/or around the eyes.

In the most preferred embodiment, the peptide or the cosmetic composition of the present invention is applied topically (preferably, in the form of a cream), more preferably, in the face of a subject (preferably, a human), even more preferably in and/or around the eyes.

In addition, in the cosmetic use of the present invention, the peptide or the composition of the present invention are used in a cosmetically effective amount. More preferably, the peptide of the present invention is used at a concentration of 0.0001% (w/v) to 0.05% (w/v). In a most preferred embodiment, the peptide of the present invention is used at a concentration of 0.0001% (w/v) to 0.001% (w/v), more preferably, 0.0005% (w/v). In another most preferred embodiment, the peptide of the present invention is used at a concentration of 0.05% (w/v) to 0.001% (w/v).

In a fifth aspect the present invention refers to a method for the cosmetic treatment of a subject in need thereof comprising the use of a peptide or a cosmetic composition of the present invention in said subject in need thereof.

The peptide and the cosmetic composition in accordance with the present invention are as explained above.

Preferably, the method is for the cosmetic treatment of cosmetic conditions related with inflammation, angiogenesis, extracellular matrix damage, oxidative stress, alterations in dermal cell junctions, apoptosis and/or hyperpigmentation, preferably cosmetic conditions related with cosmetic inflammation, cosmetic angiogenesis, cosmetic extracellular matrix damage, cosmetic oxidative stress, cosmetic alterations in dermal cell junctions, cosmetic apoptosis and/or cosmetic hyperpigmentation.

More preferably, the method is for the cosmetic treatment of skin aging signs and/or skin imperfections, more preferably, prevention, reduction and/or elimination of skin aging signs and/or skin imperfections.

As it is evident, the signs of skin aging mentioned above are cosmetic signs of skin aging and/or cosmetic skin imperfections.

Skin aging is due to chronological and/or environmental aging.

More preferably, said skin aging signs and/or skin imperfections are produced by a dysregulation of IL-33. As noted above, the peptides and cosmetic compositions of the present invention downregulate IL-33 (they downregulate the expression of IL33 gene and/or the expression of Interleukin 33).

In a most preferred embodiment, said skin aging signs and/or skin imperfections are eyebags, eyelid imperfections, wrinkles (preferably, wrinkles in the surrounding of the eyes), hyperchromia (preferably, periorbital hyperchromia) or combinations thereof.

Eyelid imperfections is preferably, saggy eyelid, more preferably, cosmetic dermatochalasis, cosmetic ptosis, cosmetic ectropion and cosmetic entropion, more preferably, cosmetic dermatochalasis, cosmetic aponeurotic ptosis, cosmetic aponeurotic blepharoptosis, cosmetic involutional ectropion and cosmetic involutional entropion.

Therefore, preferably, said skin aging signs and/or skin imperfections are eyebags, wrinkles in the surrounding of the eyes, periorbital hyperchromia, sagging eyelid or combinations thereof, more preferably, eyebags, wrinkles in the surrounding of the eyes, periorbital hyperchromia, cosmetic dermatochalasis, cosmetic aponeurotic ptosis, cosmetic aponeurotic blepharoptosis, cosmetic involutional ectropion and cosmetic involutional entropion or combinations thereof.

In the most preferred embodiment, said skin aging signs and/or skin imperfections are eyebags, wrinkles in the surrounding of the eyes, periorbital hyperchromia or combinations thereof.

In another most preferred embodiment, the method is for the cosmetic treatment of the effects in the skin of sleep deprivation, more preferably, for the cosmetic treatment of the signs of skin aging and/or skin imperfections associated with sleep deprivation, even more preferably, for the cosmetic prevention, reduction and/or elimination of the signs of skin aging and/or skin imperfections associated with sleep deprivation.

The signs of skin aging and/or skin imperfections associated with sleep deprivation are preferably sagging eyelid (as explained above), periorbital hyperchromia, signs associated with impaired barrier function or combinations thereof.

In a preferred embodiment the signs of skin aging and/or skin imperfections associated with sleep deprivation are periorbital hyperchromia.

In another preferred embodiment the signs of skin aging and/or skin imperfections associated with sleep deprivation are signs associated with impaired barrier function, more preferably altered skin permeability, skin inflammation, lack of cellular cohesion in the skin or combinations thereof, even more preferably eyebags, wrinkles (preferably, wrinkles in the surrounding of the eyes) or combinations thereof.

Also, in a preferred embodiment, the subject in need thereof is a mammal, even more preferably, a human.

Preferably, in the method for the cosmetic treatment of the present invention, the use of the peptide or of the cosmetic composition of the present invention in the subject in need thereof is by means of the application of said peptide or cosmetic composition of the present invention in the subject in need thereof.

It is contemplated that the peptide or the cosmetic composition of the present invention is applied by means of iontophoresis, more preferably in the face of the subject (preferably, a human), even more preferably in and/or around the eyes.

It is also contemplated that the peptide or the cosmetic composition of the present invention is applied subcutaneously, more preferably, in the face of the subject (preferably, a human), even more preferably in and/or around the eyes.

In the most preferred embodiment, the peptide or the cosmetic composition of the present invention is applied topically (preferably, in the form of a cream), more preferably, in the face of the subject (preferably, a human), even more preferably in and/or around the eyes.

In addition, in the cosmetic method of the present invention, the peptide or the composition of the present invention are used in a cosmetically effective amount. More preferably, the peptide of the present invention is used at a concentration of 0.0001% (w/v) to 0.05% (w/v). In a most preferred embodiment, the peptide of the present invention is used at a concentration of 0.0001% (w/v) to 0.001 % (w/v), more preferably, 0.0005% (w/v). In another most preferred embodiment, the peptide of the present invention is used at a concentration of 0.05% (w/v) to 0.001% (w/v).

In a sixth aspect, the present invention refers to a peptide or a pharmaceutical composition of the present invention for use as a medicament.

The peptide and pharmaceutical composition in accordance with the present invention are as explained above.

Preferably, the peptide or composition of the present invention is used in a therapeutically effective amount.

In a preferred embodiment, the peptides and compositions of the present invention are for use in a mammal, more preferably in a human.

Preferably, the peptide or pharmaceutical composition of the present invention is for use in the treatment of a medical conditions related with inflammation, angiogenesis, extracellular matrix damage, oxidative stress, alterations in dermal cell junctions, apoptosis and/or hyperpigmentation, diseases related with lipid peroxidation, diseases related with protein glycation or combinations thereof.

Preferably, the diseases related with lipid peroxidation is atherosclerosis, Inflammatory Bowel Disease, Retinopathy of Prematurity, Borderline Personality Disorder, Asthma, Parkinson's disease and kidney damage.

Preferably, the diseases related with protein glycation is diabetes (more preferably, diabetic vasculopathy), renal failure and age-related degenerative diseases.

In a most preferred embodiment, the peptide or pharmaceutical composition of the present invention is for use in the treatment of eyelid imperfections, preferably extensive sagging eyelid, wherein said extensive sagging eyelid leads to visual field loss, ocular or eyelid irritation and/or headaches; more preferably, dermatochalasis, ptosis, ectropion and entropion; even more preferably, dermatochalasis, aponeurotic ptosis, aponeurotic blepharoptosis, involutional ectropion and involutional entropion.

It is contemplated that the peptide or the pharmaceutical composition of the present invention is applied by means of iontophoresis, more preferably in the face of the subject (preferably, a human), even more preferably in and/or around the eyes.

It is also contemplated that the peptide or the pharmaceutical composition of the present invention is applied subcutaneously, more preferably, in the face of the subject (preferably, a human), even more preferably in and/or around the eyes.

In the most preferred embodiment, the peptide or the pharmaceutical composition of the present invention is applied topically (preferably, in the form of a cream), more preferably, in the face of the subject (preferably, a human), even more preferably in and/or around the eyes.

To allow a better understanding, the present invention is described in more detail below with reference to the enclosed drawings, which are presented by way of example, and with reference to illustrative and non-limitative examples.

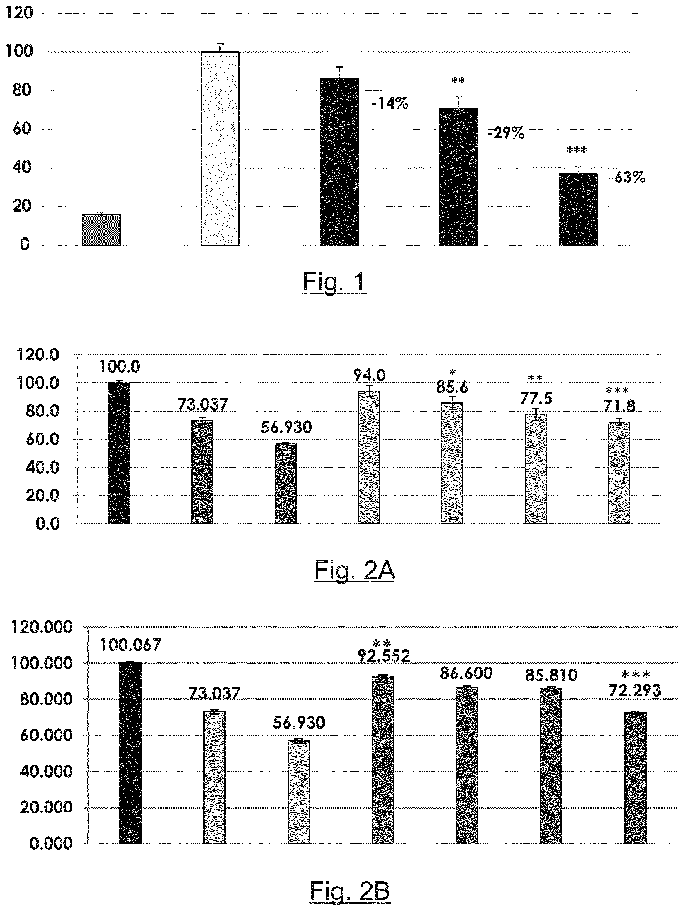

Figure 1 shows the IL-33 protein levels in human dermal fibroblasts adult cells treated with peptide Ac-SEQ ID NO:1-NH₂. Results are shown as percentage in comparison with the positive control (treatment with 300 U/mL of Interferon-γ), which is stablished as 100%. The columns in the x-axis, from left to right refer to: basal state of the cells, positive control and cells treated with 0.005 mg/mL, 0.01 mg/mL or 0.05 mg/mL of Ac-SEQ ID NO:1-NH₂, respectively. Samples treated with the peptide of the present invention were also treated with 300 U/mL of Interferon-γ. The y-axis refers to the % of IL-33 in pg/mL, with regard to the positive control that is stablished as 100%. ** refers to a p < 0.01; and *** refers to a p < 0.001.

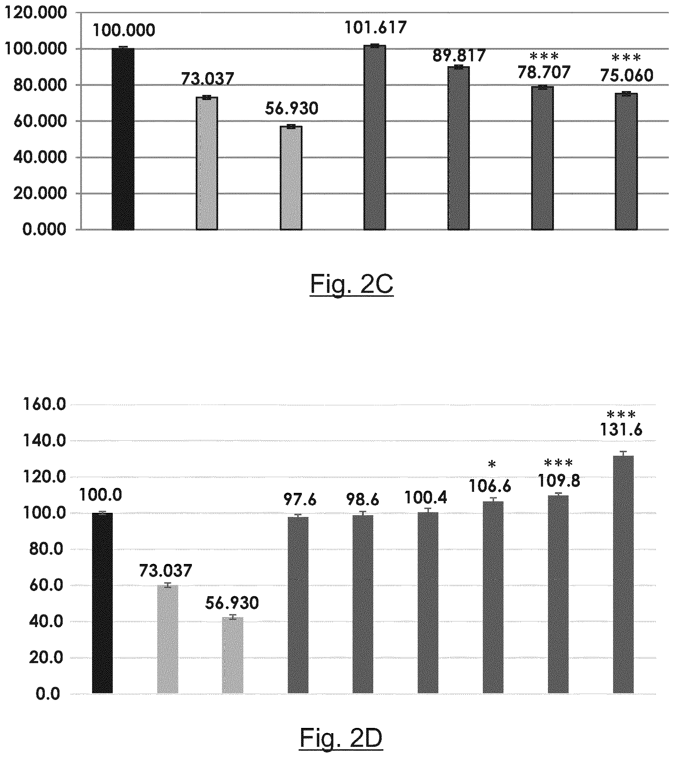

Figure 2 shows the results for the inhibition of mushroom tyrosinase activity obtained in example 11. Tyrosinase activity with regard to the control (without peptide), which is stablished as 100%, is measured. Figure 2A refers to the results of tyrosinase activity obtained with Ac-SEQ ID NO:1-NH₂ and, hence, columns in the x-axis, from left to right, refer to: control wells and wells containing 400 µM and 800 µM of kojic acid and wells containing 0.05 mg/mL, 0.25 mg/mL, 0.5 mg/mL and 2.5 mg/mL of Ac-SEQ ID NO:1-NH₂, respectively. Figure 2B refers to the results of tyrosinase activity obtained with Ac-SEQ ID NO:2-NH₂ and, hence, columns in the x-axis, from left to right, refer to: control wells, wells containing 400 µM and 800 µM of kojic acid and wells containing 0.05 mg/mL, 0.25 mg/mL, 0.5 mg/mL and 2.5 mg/mL of Ac-SEQ ID NO:2-NH₂, respectively. Figure 2C refers to the results of tyrosinase activity obtained with Ac-SEQ ID NO:3-NH₂ and, hence, columns in the x-axis, from left to right, refer to: control wells, wells containing 400 µM and 800 µM of kojic acid and wells containing 0.05 mg/mL, 0.25 mg/mL, 0.5 mg/mL and 2.5 mg/mL of Ac-SEQ ID NO:3-NH₂, respectively. Figure 2D refers to the results obtained with Ac-SEQ ID NO: 4-NH₂ and, hence, columns in the x-axis, from left to right, refer to: control wells and wells treated with 0.005 mg/mL, 0.025 mg/mL, 0.05 mg/mL, 0.25 mg/mL, 0.5 mg/mL and 2.5 mg/mL of Ac-SEQ ID NO:4-NH₂, respectively. In figures 2A, 2B, 2C and 2D, the y-axis refers to the percentage of tyrosinase activity, stablishing as 100% the tyrosinase activity of the control. * refers to a p < 0.05; ** refers to a p < 0.01; and *** refers to a p < 0.001.

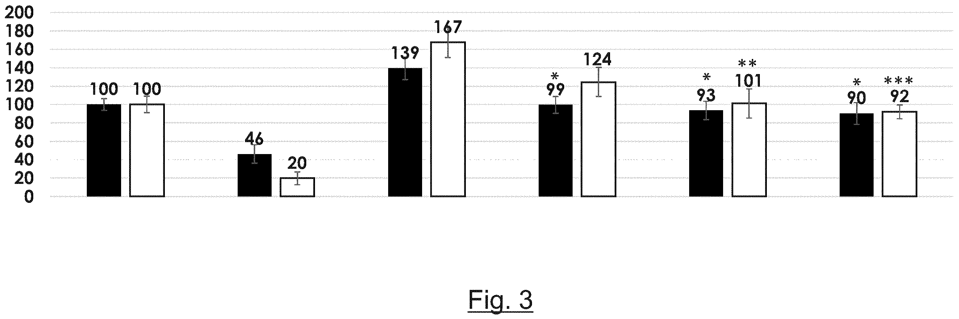

Figure 3 shows the tube formation (angiogenesis) in HUVEC treated with Ac-SEQ ID NO:1-NH₂ in example 12. More precisely, columns in black refer to the number of meshes as percentage in comparison with the basal state (stablishing the basal state as 100%); and columns in white refer to the total area of meshes as percentage in comparison with the basal state (stablishing the basal state as 100%). In addition, each group of two columns (one black and one white) from left to right in the x-axis in this figure correspond to: basal; negative control (treatment with 20 µM of Suramin); positive control (treatment with 10 ng/mL IL-33); treatment with 0.01 mg/mL of Ac-SEQ ID NO:1-NH₂ and 10 ng/mL IL-33; treatment with 0.05 mg/mL of Ac-SEQ ID NO:1-NH₂ and 10 ng/mL IL-33; and treatment with 0.1 mg/mL of Ac-SEQ ID NO:1-NH₂ and 10 ng/mL IL-33. The y-axis refers to the percentage of number of meshes (for black columns) or of total area of meshes (for white columns) (depending on the column considered) in comparison with the basal state (stablished as 100%). * refers to a p < 0.05; ** refers to a p < 0.01; and *** refers to a p < 0.001.

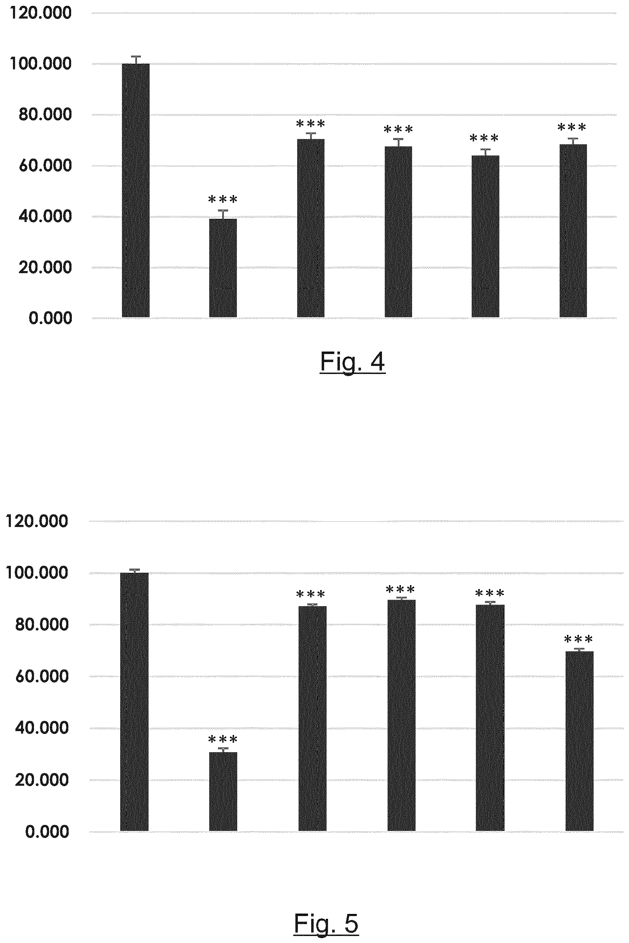

Figure 4 shows the results of example 13, this is the peroxidation of lipids in comparison with the negative control, which is stablished as 100%. In the x-axis, columns from left to right correspond to: negative control (lipid substrate without antioxidant compound), positive control (lipid substrate treated with 250 µg/mL of Trolox (a stable vitamin E analogue) but not with a peptide of the present invention) and treatment with 0.01 mg/mL, 0.05 mg/mL, 0.1 mg/mL or 0.5 mg/mL of Ac-SEQ ID NO:1-NH₂, respectively. All samples had a lipid substrate and all samples except the negative control also comprised a lipid peroxidant (thiobarbituric acid). A 2-hour incubation at 37°C was performed before reading the plate at Excitation at: 500 nm; Emission at: 530nm.The y-axis refers to the percentage of lipid peroxidation in comparison with the negative control, which is stablished as 100 %. * refers to a p < 0.05; ** refers to a p < 0.01; and *** refers to a p < 0.001.

Figure 5 shows the results of example 14, this is the glycation of proteins in comparison with the negative control, which is stablished as 100%. In the x-axis, columns from left to right correspond to: negative control (sugar substrate), positive control (sugar substrate treated with 690 µg/mL of aminoguanidine (AG, a representative antiglycation compound)) and treatment with 0.01 mg/mL, 0.05 mg/mL, 0.1 mg/mL or 0.5 mg/mL of Ac-SEQ ID NO:1-NH₂, respectively. All samples had a sugar substrate and all samples except the negative control also comprised a glycation agent. A 72-hour incubation at 37°C was performed before reading the plate at Excitation at: 370 nm; Emission at: 440 nm. The y-axis refers to the percentage of protein glycation in comparison with the negative control, which is stablished as 100%. * refers to a p < 0.05; ** refers to a p < 0.01; and *** refers to a p < 0.001.

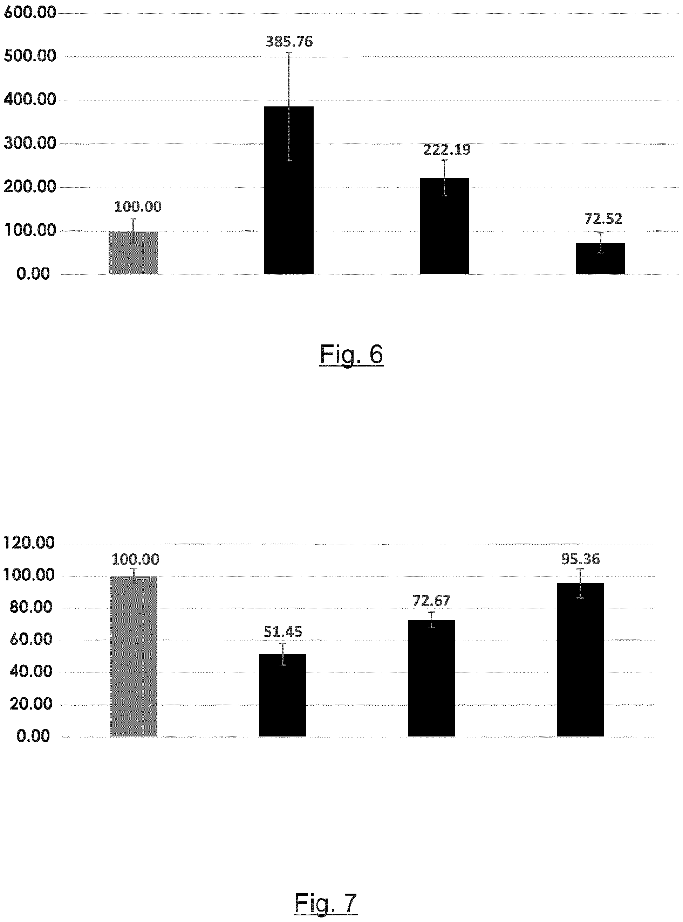

Figure 6 shows the *ex-vivo* results of example 14, this is the protective effect of Ac-SEQ ID NO:1-NH₂ in front methylglyoxal (MG)-induced glycation in comparison with the negative control (untreated explants), which is stablished as 100%. In the x-axis, columns from left to right correspond to: negative control (untreated explants), positive control (MG, not treated with any cream), and MG with placebo cream or cream containing 1% (w/v) of Ac-SEQ ID NO:1-NH₂, respectively. The y-axis refers to the percentage of CML staining (N(ε)-carboxymethyl-Lysine) in comparison with the negative control, which is stablished as 100%.

Figure 7 shows the ex-vivo results of example 15, this is the protective effect of Ac-SEQ ID NO:1-NH₂ in front methylglyoxal (MG)-induced fibrillin-1 degradation in comparison with the negative control (untreated explants), which is stablished as 100%. In the x-axis, columns from left to right correspond to: negative control (untreated explants), positive control (MG, not treated with any cream), and MG with placebo cream or cream containing 1% (w/v) of Ac-SEQ ID NO:1-NH₂, respectively. The y-axis refers to the percentage of fibrillin-1 staining in comparison with the negative control, which is stablished as 100%.

### EXAMPLES

### Abbreviations:

The abbreviations used for amino acids follow the 1983 IUPAC-IUB Joint Commission on Biochemical Nomenclature recommendations outlined in Eur. J. Biochem. (1984) 138:937.

Ac, acetyl; Arg, arginine; Boc, tert-butyloxycarbonyl; C-terminal, carboxy-terminal; DCM, dichloromethane; DIEA, N,N'-diisopropylethylamine; DIPCDI, N,N'-diisopropylcarbodiimide; DMF, N,N-dimethylformamide; equiv, equivalent; ESI-MS, electrospray ionization mass spectrometry; Fmoc, 9-fluorenylmethyloxycarbonyl; Glu, Glutamic acid; Gln, Glutamine; hiPSC, human induced pluripotent stem cells; HOBt, 1-hydroxybenzotriazole; HPLC, high performance liquid chromatography; HRP, Horseradish peroxidase; INCI, International Nomenclature of Cosmetic Ingredients; MBHA, p-methylbenzhydrylamine; Me, methyl; MeCN, acetonitrile; MeOH, methanol; Met, Methionine; N-terminal, amino-terminal; Palm, palmitoyl; Pbf, 2,2,4,6,7-pentamethyldihydrobenzofuran-5-sulfonyl; PFA, paraformaldehyde; PMA, phorbol 12-myristate 13-acetate; PMSF, Phenylmethanesulfonyl; RT, room temperature; tBu, *tert-*butyl; TFA, trifluoroacetic acid; TIS, triisopropylsilane; TMB, Tetramethylbenzidine; Trt, triphenylmethyl or trityl.

Regarding the chemical synthesis procedures included in the examples, it is noted that all synthetic processes were carried out in polypropylene syringes fitted with porous polyethylene discs or Pyrex^{®} reactors fitted with porous plates. All the reagents and solvents were synthesis quality and were used without any additional treatment. The solvents and soluble reagents were removed by suction. The Fmoc group was removed with piperidine-DMF (2:8, v/v) (at least 1×1 min, 2×10 min, 5 mL/g resin) (Lloyd Williams P. et al., Chemical Approaches to the Synthesis of Peptides and Proteins, CRC, 1997, Boca Raton (Fla., USA)). Washes between stages of deprotection, coupling, and, again, deprotection, were carried out with DMF (3×1 min) and DCM (3×1 min) each time using 10 ml solvent/g resin. Coupling reactions were performed with 3 ml solvent/g resin. The control of the couplings was performed by carrying out the ninhydrin test (Kaiser E. et al., Anal. Biochem., 1970, 34: 595598). All synthetic reactions and washes were carried out at RT.

### Example 1. Synthesis and preparation of the peptides.

- Obtaining Fmoc-AA₁-AA₂-AA₃-AA₄-AA₅-AA₆-Rink-MBHA-resin, wherein AA₁ is L-Arg; AA₂ is L-Arg; AA₃ is L-Gln; AA₄ is L-Met; AA₅ is L-Arg; and AA₆ is L-Glu.

Weights were normalized. 4.8 g (2.5 mmol) of the Fmoc-Rink-MBHA resin with a functionalization of 0.52 mmol/g were treated with piperidine-DMF according to the described general protocol known in the state of the art in order to remove the Fmoc group. 3.19 g of Fmoc-L-Glu(OtBu)-OH (7.5 mmol; 3 equiv) were incorporated onto the deprotected resin in the presence of DIPCDI (1.17 mL; 7.5 mmol; 3 equiv) and HOBt (1.01 g; 7.5 mmol; 3 equiv) using DMF as a solvent for one hour.

The resin was then washed as described in the general methods known in the state of the art and the deprotection treatment of the Fmoc group was repeated to couple the next amino acid. Following the previously described protocols 4.86 g of Fmoc-L-Arg(Pbf)-OH (7.5 mmol; 3 equiv); subsequently 2.78 g of Fmoc-L-Met-OH (7.5 mmol; 3 equiv); subsequently 4.58 g of Fmoc-L-Gln(Trt)-OH (7.5 mmol; 3 equiv); subsequently 4.86 g Fmoc-L-Arg(Pbf)-OH (7.5 mmol; 3 equiv) and subsequently 4.86 g of Fmoc-L-Arg(Pbf)-OH (7.5 mmol; 3 equiv) were coupled, sequentially, each coupling in the presence of 1.01 g of HOBt (7.5 mmol; 3 equiv) and 1.17 mL of DIPCDI (7.5 mmol; 3 equiv). As already noted above, between each amino acid addition step, a deprotection treatment of the Fmoc group was performed.

After the synthesis, the peptide resins were washed with DCM (5 times for 3 minutes each one) and dried under vacuum.
- Obtaining Fmoc-AA₁-AA₂-AA₃-AA₄-AA₅-AA₆-Rink-MBHA-resin, wherein AA₁ is L-Met; AA₂ is L-Arg; AA₃ is L-Arg; AA₄ is L-Glu; AA₅ is L-Gln; and AA₆ is L-Arg.

Weights were normalized. 4.8 g (2.5 mmol) of the Fmoc-Rink-MBHA resin with a functionalization of 0.52 mmol/g were treated with piperidine-DMF according to the described general protocol known in the state of the art in order to remove the Fmoc group. 4.86 g of Fmoc-L-Arg(Pbf)-OH (7.5 mmol; 3 equiv) were incorporated onto the deprotected resin in the presence of DIPCDI (1.17 mL; 7.5 mmol; 3 equiv) and HOBt (1.01 g; 7.5 mmol; 3 equiv) using DMF as a solvent for one hour.

The resin was then washed as described in the general methods known in the state of the art and the deprotection treatment of the Fmoc group was repeated to couple the next amino acid. Following the previously described protocols 4.58 g of Fmoc-L-Gln(Trt)-OH (7.5 mmol; 3 equiv); subsequently 3.19 g of Fmoc-L-Glu(OtBu)-OH (7.5 mmol; 3 equiv); subsequently 4.86 g of Fmoc-L-Arg(Pbf)-OH (7.5 mmol; 3 equiv); subsequently 4.86 g Fmoc-L-Arg(Pbf)-OH (7.5 mmol; 3 equiv) and subsequently 2.78 g of Fmoc-L-Met-OH (7.5 mmol; 3 equiv) were coupled, sequentially, each coupling in the presence of 1.01 g of HOBt (7.5 mmol; 3 equiv) and 1.17 mL of DIPCDI (7.5 mmol; 3 equiv). As already noted above, between each amino acid addition step, a deprotection treatment of the Fmoc group was performed.

After the synthesis, the peptide resins were washed with DCM (5 times for 3 minutes each one) and dried under vacuum.
- Obtaining Fmoc-AA₁-AA₂-AA₃-AA₄-AA₅-AA₆-Rink-MBHA-resin, wherein AA₁ is L-Arg; AA₂ is L-Glu; AA₃ is L-Gln; AA₄ is L-Met; AA₅ is L-Arg; and AA₆ is L-Arg.

Weights were normalized. 4.8 g (2.5 mmol) of the Fmoc-Rink-MBHA resin with a functionalization of 0.52 mmol/g were treated with piperidine-DMF according to the described general protocol known in the state of the art in order to remove the Fmoc group. 4.86 g of Fmoc-L-Arg(Pbf)-OH (7.5 mmol; 3 equiv) were incorporated onto the deprotected resin in the presence of DIPCDI (1.17 mL; 7.5 mmol; 3 equiv) and HOBt (1.01 g; 7.5 mmol; 3 equiv) using DMF as a solvent for one hour.

The resin was then washed as described in the general methods known in the state of the art and the deprotection treatment of the Fmoc group was repeated to couple the next amino acid. Following the previously described protocols 4.86 g of Fmoc-L-Arg(Pbf)-OH (7.5 mmol; 3 equiv); subsequently 2.78 g of Fmoc-L-Met-OH (7.5 mmol; 3 equiv); subsequently 4.58 g of Fmoc-L-Gln(Trt)-OH (7.5 mmol; 3 equiv); subsequently 3.19 g Fmoc-L-Glu(OtBu)-OH (7.5 mmol; 3 equiv) and subsequently 4.86 g of Fmoc-L-Arg(Pbf)-OH (7.5 mmol; 3 equiv) were coupled, sequentially, each coupling in the presence of 1.01 g of HOBt (7.5 mmol; 3 equiv) and 1.17 mL of DIPCDI (7.5 mmol; 3 equiv). As already noted above, between each amino acid addition step, a deprotection treatment of the Fmoc group was performed.

After the synthesis, the peptide resins were washed with DCM (5 times for 3 minutes each one) and dried under vacuum.

**Example 2.** Removal of Fmoc N-Terminal protective group of the peptides synthesized in accordance with Example 1.

The N-terminal Fmoc group of the peptidyl resins obtained in Example 1 was deprotected with 20% (volume/volume, hereinafter v/v) piperidine in DMF (1×1 min+2×10 min) (Lloyd Williams P. et al. (1997) Chemical Approaches to the Synthesis of Peptides and Proteins. CRC, Boca Raton (Fla., USA)). The peptidyl resins were washed with DMF (5×1 min), DCM (4×1 min), and dried under vacuum.

**Example 3.** Process for introducing the R₁ Acetyl group onto the peptidyl resins obtained in accordance with Example 2.

1 mmol (1 equiv) of the peptidyl resins obtained in accordance with Example 2 was treated with 25 equivalents of acetic anhydride in the presence of 25 equivalents of DIEA using 5 mL of DMF as a solvent. They were left to react for 30 minutes, after which the peptidyl resins were washed with DMF (5×1 min), DCM (4×1 min), and were dried under vacuum.

**Example 4.** Cleavage process from the polymeric support of the peptidyl resins obtained in accordance with Example 2 and 3.

Weights were normalized. 200 mg of the dried peptidyl resin obtained in any of Examples 2 or 3 were treated with 5 mL of TFA/TIS/H₂O (90:5:5) for 2 hours at room temperature under stirring. The filtrates were collected and precipitated using 50 mL (8 to 10-fold) of cold diethyl ether. The ethereal solutions were evaporated to dryness at reduced pressure and room temperature, the precipitates were redissolved in 50% (v/v) MeCN in H₂O and lyophilized.

**Example 5.** Characterization of the peptides synthesized and prepared in accordance with example 4.

HPLC analysis of the peptides obtained in accordance with example 4 was carried out with a Shimadzu equipment (Kyoto, Japan) using a reverse-phase column (150x4.6 mm, XBridge Peptide BEH C18, 3.5 µm, Waters, USA) in gradients of MeCN (+0.036% (v/v) TFA) in H₂O (+0.045% (v/v) TFA) at a flow rate of 1.25 mL/min and detection was carried out at 220 nm. All peptides showed a purity exceeding 80%. The identity of the peptides obtained was confirmed by ESI-MS in a Water ZQ 4000 detector using MeOH as the mobile phase and a flow rate of 0.2 mL/min. Results obtained demonstrated that peptides Ac-SEQ ID NO: 1- NH₂, Ac-SEQ ID NO: 2- NH₂ and Ac-SEQ ID NO: 3- NH₂ were correctly and effectively synthesized.

**Example 6.** Preparation of a cosmetic facial composition containing Ac-SEQ ID NO: 1-NH₂.

A cosmetic facial composition in accordance with table 1 below was prepared. To that end, components from phase A were dissolved in a suitable vessel and the mixture was heated to 70-75°C. In another vessel the components of phase B were mixed together and the mixture was heated to 70-75°C. Next, phase C (TEGOLON 12-10 (INCl: Nylon-12)) was slowly added to phase B, under stirring, until it was completely dissolved. The mixture was heated to 70-75°C. Next the solution of phase A was added to the mixture of phases B and C under turbine stirring to form an emulsion. Next, phase D was slowly added to the mixture, maintaining the stirring until an homogeneous emulsion was obtained. Finally, with the mixture at about 30°C, the commercial formulation containing the compound Ac-SEQ ID NO: 1-NH₂ (INCl: Water (Aqua), Glycerin, Caprylyl Glycol, Ac-SEQ ID NO: 1-NH₂) was slowly added maintaining stirring.

**Table 1. Cosmetic facial composition prepared in Example 6.**

| Phase | Ingredient | % in weight (g/100g) |
|---|---|---|
| A | Water | q. s. (quantum satis) 100 |
| A | Disodium EDTA | 0.15 |
| A | Magnesium sulfate | 1.5 |
| A | Glycerin | 2.5 |
| B | Caprylic/Capric triglyceride | 8 |
| B | Isononyl/Isononanoate | 15 |
| B | Polyglyceryl-4 diisostearate/polyhydroxystearate/sebacate | 3 |
| B | Verstatil TBO (INCl: Triethyl citrate, Caprylyl glycol, Benzoic acid): | |
| | Thriethyl citrate | 0.455 |
| | Caprylyl glycol | 0.37 |
| | Benzoic acid | 0.175 |
| B | Synthetic beeswax | 3 |
| C | Nylon-12 | 2 |
| D | BRB SG 516 (INCI: Dimethicone, Dimethicone/Vinyl dimethicone crosspolymer): | |
| | Dimethicone | 1.7 |
| | Dimethicone/Vinyl dimethicone crosspolymer | 0.3 |
| E | Commercial formulation of Ac-SEQ ID NO: 1- NH₂ (INCI: Water (Aqua), Glycerin, Caprylyl glycol, Ac-SEQ ID NO:1-NH₂): | |
| | Water (Aqua) | 0.9445 |
| | Glycerin | 0.05 |
| | Caprylyl glycol | 0.005 |
| | Ac-SEQ ID NO: 1-NH₂ | 0.0005 |

**Example 7.** Analysis of IL33 expression modulation in HEKa (Human Epidermal Keratinocytes, adult) and HDFa (Human Dermal Fibroblasts, adult) cells Peptides Ac-SEQ ID NO: 1- NH₂, Ac-SEQ ID NO: 2- NH₂ and Ac-SEQ ID NO: 3- NH₂ were analysed for its capacity to modulate expression of IL33 in HEKa cells (all three peptides) and in HDFa (only analysed for peptide Ac-SEQ ID NO: 1- NH₂).

The peptides used in this example were prepared in accordance with examples 1 to 5.

A stock solution of the peptide was prepared in water at 1 mg/mL. Working solution was freshly prepared at the specified concentration from stock solution in the corresponding supplemented medium.

Untreated cells were used as basal or negative control samples.

RNA (ribonucleic acid) extraction and RT-qPCR (reverse transcription quantitative polymerase chain reaction) were performed. Briefly, HDFa and HEKa cells were seeded in duplicate (n=2) in 6-well plates at a density of 4 × 10⁵ cells/well and maintained at standard culture conditions (106 Medium supplemented with 1% (v/v) LSGS for HDFa cells; Medium Epilife supplemented with 1% (v/v) EDGS and 1% (v/v) penicillin/streptomycin for HEKa cells; 37°C, 95% room humidity, 5% CO₂) for 24 hours. Then, cells were treated with peptide Ac-SEQ ID NO: 1-NH₂, Ac-SEQ ID NO: 2- NH₂ or Ac-SEQ ID NO: 3- NH₂ at the concentration of 0.01 mg/mL for 24 hours. Untreated cells were used as basal or negative control.

Cells were finally lysed, and replicates were pooled together for RNA extraction using Qiagen RNeasy Mini kit following manufacturer's instructions. Purified RNAs were used to generate the corresponding cDNAs (complementary deoxyribonucleic acids) by reverse transcription using a commercial kit High capacity cDNA reverse transcription kit (Applied Biosystems) which served as templates for amplification. RT-qPCR was performed with the appropriate TaqMan assay probes for IL33 (plus GAPDH - Glyceraldehyde 3-phosphate dehydrogenase- that was used as housekeeping gene) and 2x gene expression Master Mix using StepOne plus Real-Time PCR instrument. Amplification included 40 cycles of: 15 seconds at 95°C (denaturation) and 1 minute at 60°C (Annealing and extension) (Arya, M., Shergill, I.S., Williamson, M., Gommersall, L., Arya, N., Patel, H.R. (2005) Basic principles of real-time quantitative PCR. Expert Rev. Mol. Diagn.; 5(2):209-19; and Jozefczuk, J. and Adjaye, J. (2011) Quantitative real-time PCR-based analysis of gene expression. Methods Enzymol. 500; 99-109).

The obtained data was analysed using the ΔΔCt method, which provides the target gene expression values as fold changes in the treated sample compared with an untreated basal sample. Both samples were normalized with the relative expression of a housekeeping gene GAPDH (Glyceraldehyde 3-phosphate dehydrogenase).

The steps for analysis included:
1. Calculate the average Ct for each condition
2. Calculate the ΔCT test sample and the ΔCT untreated sample
3. Calculate the ΔΔCT: ΔΔCT = ΔCT test sample - ΔCT untreated sample
4. Obtain ratio by 2^{- ΔΔCT}

In this assay, a clear downregulation of the expression of IL33 gene was observed in HEKa cells for all the peptides tested:
- Ac-SEQ ID NO: 1- NH₂: -4.58 fold change in treated cells with regard to basal control.
- Ac-SEQ ID NO: 2- NH₂: -3.77 fold change in treated cells with regard to basal control.
- Ac-SEQ ID NO: 3- NH₂: -3.93 fold change in treated cells with regard to basal control.

In addition, for peptide Ac-SEQ ID NO: 1- NH₂ a downregulation of the expression of IL33 was also observed in HDFa cells (-1.22 fold change treated cells with regard to basal control).

The results of the present example demonstrate the usefulness of the peptides of the present invention to downregulate the expression of IL33, thus, as explained above, providing for an improvement mainly in inflammation, and additionally in the skin barrier, a decrease in angiogenesis and a decrease in pigmentation. Therefore, the results of this example demonstrate the usefulness of the peptides of the present invention to prevent, reduce and or eliminate signs of skin aging and/or skin imperfections, more preferably, eyebags, eyelid imperfections, wrinkles in the surrounding of the eyes and dark circles (periorbital hyperchromia).

**Example 8.** Analysis of the production of protein IL-33 in HDFa cells.

Peptide Ac-SEQ ID NO: 1- NH₂ was analysed for its capacity to modulate IL-33 production in adult human dermal fibroblasts.

Peptide Ac-SEQ ID NO: 1-NH₂ was prepared in accordance with examples 1 to 5.

Adult human dermal fibroblasts (HDFa) were seeded on 24-well plate at concentration of 1 ×10⁵ cells/mL in their corresponding growth media and incubated for 24 hours in standard culture conditions; at that time, three different concentrations (0.005, 0.01 and 0.05 mg/mL) of the Ac-SEQ ID NO: 1- NH₂ were added in presence of IL-33-induction stimulus (IFNγ, 300 U/mL). Cells without stimuli were also included as basal group. Positive control group was cells treated with 300 U/mL of IFNγ, but without being treated with a peptide of the present invention. 24 hours later, conditioned media was collected, and cells processed for protein extraction.

IL-33 levels in the cell lysates were analysed with a commercial ELISA kit (DuoSet ELISA. R&D). Absorbances were measured with an automated spectrophotometer plate-reader.

Results obtained in this example appear summarized in figure 1, wherein it can be seen that IFNγ (300 U/mL) induces an increase in the production of protein IL-33 in HDFa cells and that peptide Ac-SEQ ID NO: 1- NH₂ reduces the IL-33 protein levels produced by HDFa cells exposed to IFNγ (300 U/mL) in a dose-dependent manner (this is, a greater reduction of IL-33 protein levels is observed as concentration of peptide Ac-SEQ ID NO: 1- NH₂ is increased).

The results of the present example demonstrate the usefulness of the peptides of the present invention to reduce inflammation through the downregulation of the expression of IL-33, thus, as explained above, providing for an improvement in the skin barrier, a decrease in angiogenesis and a decrease in pigmentation. Therefore, the results of this example demonstrate the usefulness of the peptides of the present invention to prevent, reduce and or eliminate signs of skin aging and/or skin imperfections, more preferably, eyebags, eyelid imperfections, wrinkles in the surrounding of the eyes and dark circles (periorbital hyperchromia).

**Example 9.** Analysis of the gene expression modulation in human epidermal keratinocytes, adult (hereinafter, HEKa).

Peptides Ac-SEQ ID NO: 1- NH₂, Ac-SEQ ID NO: 2- NH₂ and Ac-SEQ ID NO: 3- NH₂ were analysed for its capacity to modulate the expression of genes related epidermal cell cohesion (see table 2 for the analysed genes).

The peptides used in this example were prepared in accordance with examples 1 to 5.

A stock solution of the peptide was prepared in water at 1 mg/mL. Working solution was freshly prepared at the specified concentration from stock solution in the corresponding supplemented medium.

Untreated cells were used as basal or negative control samples.

RNA (ribonucleic acid) extraction and RT-qPCR (reverse transcription quantitative polymerase chain reaction) were performed. Briefly, HEKa cells were seeded in duplicate (n=2) in 6-well plates at a density of 4 × 10⁵ cells/well and maintained at standard culture conditions (Medium Epilife supplemented with 1% (v/v) EDGS and 1% (v/v) penicillin/streptomycin; 37°C, 95% room humidity, 5% CO₂) for 24 hours.

Then, cells were treated with peptide Ac-SEQ ID NO: 1-NH₂, Ac-SEQ ID NO: 2- NH₂ or Ac-SEQ ID NO: 3- NH₂ at the concentration of 0.01 mg/mL for 24 hours. Untreated cells were used as basal or negative control.

Cells were finally lysed, and replicates were pooled together for RNA extraction using Qiagen RNeasy Mini kit following manufacturer's instructions. Purified RNAs were used to generate the corresponding cDNAs (complementary deoxyribonucleic acids) by reverse transcription using a commercial kit High capacity cDNA reverse transcription kit (Applied Biosystems) which served as templates for amplification. RT-qPCR was performed with the panel of appropriate TaqMan assay probes for genes shown in table 1 (plus GAPDH -Glyceraldehyde 3-phosphate dehydrogenase- that was used as housekeeping gene) and 2x gene expression Master Mix using StepOne plus Real-Time PCR instrument. Amplification included 40 cycles of: 15 seconds at 95°C (denaturation) and 1 minute at 60°C (Annealing and extension) (Arya, M., Shergill, I.S., Williamson, M., Gommersall, L., Arya, N., Patel, H.R. (2005) Basic principles of real-time quantitative PCR. Expert Rev. Mol. Diagn.; 5(2):209-19; and Jozefczuk, J. and Adjaye, J. (2011) Quantitative real-time PCR-based analysis of gene expression. Methods Enzymol. 500; 99-109).

**Table 2. Genes analysed in example 9 (abbreviation and complete name).**

| Abbreviation | Gene |
|---|---|
| JUP | Junction Plakoglobin |
| OCLN | Occludin |
| DSP | Desmoplakin |
| CLDN1 | Claudin 1 |
| CLDN4 | Claudin 4 |
| DSC2 | Desmocollin 2 |
| CDSN | Corneodesmosin |
| PCDH1 | Protocadherin 1 |
| TJP1 | Tight Junction Protein 1 |
| PKP2 | Plakophilin 2 |
| PPL | Periplakin |

The obtained data was analysed using the ΔΔCt method, which provides the target gene expression values as fold changes in the treated sample compared with an untreated basal sample. Both samples were normalized with the relative expression of a housekeeping gene GAPDH (Glyceraldehyde 3-phosphate dehydrogenase).

The steps for analysis included:
1. Calculate the average Ct for each condition
2. Calculate the ΔCT test sample and the ΔCT untreated sample
3. Calculate the ΔΔCT: ΔΔCT = ΔCT test sample - ΔCT untreated sample
4. Obtain ratio by 2^{- ΔΔT}

The results of this assay appear also summarized in tables 3 to 5.

**Table 3. Fold change between treated cells and basal control obtained in HEKa cells treated with 0.01 mg/mL of Ac-SEQ ID NO: 1-NH₂ for 24 hours.**

| Abbreviation | Ac-SEQ ID NO: 1-NH₂ (Fold change) |
|---|---|
| JUP | 1.42 |
| OCLN | 1.98 |
| DSP | 1.28 |
| CLDN1 | 1.57 |
| CLDN4 | 2.64 |
| DSC2 | 1.23 |
| CDSN | 1.46 |
| PCDH1 | 2.41 |
| TJP1 | 1.25 |
| PKP2 | 1.72 |
| PPL | 1.54 |

**Table 4. Fold change between treated cells and basal control obtained in HEKa cells treated with 0.01 mg/mL of Ac-SEQ ID NO: 2-NH₂ for 24 hours.**

| Abbreviation | Ac-SEQ ID NO: 2- NH₂ (Fold change) |
|---|---|
| OCLN | 1.58 |
| CLDN4 | 1.51 |
| PCDH1 | 2.14 |
| TJP1 | 1.34 |
| PKP2 | 1.42 |

**Table 5. Fold change between treated cells and basal control obtained in HEKa cells treated with 0.01 mg/mL of Ac-SEQ ID NO: 3-NH₂ for 24 hours.**

| Abbreviation | Ac-SEQ ID NO: 3- NH₂ (Fold change) |
|---|---|
| JUP | 1.23 |
| OCLN | 1.99 |
| DSP | 1.28 |
| CLDN1 | 1.5 |
| DSC1 | 1.41 |
| CLDN4 | 1.90 |
| DSC2 | 1.43 |
| PCDH1 | 2.48 |
| TJP1 | 1.43 |
| PKP2 | 1.86 |
| PPL | 1.52 |

As can be readily derived from tables 3 to 5:
- Peptide Ac-SEQ ID NO: 1- NH₂ clearly upregulated key genes related to tight junctions and barrier function: JUP, OCLN, DSP, CLDN1, CLDN4, DSC2, CDSN, PCDH1, TJP1, PKP2 and PPL.
- Peptide Ac-SEQ ID NO: 2- NH₂ clearly upregulated key genes related to tight junctions and barrier function: OCLN, CLDN4, PCDH1, TJP1 and PKP2.
- Peptide Ac-SEQ ID NO: 3- NH₂ clearly upregulated key genes related to tight junctions and barrier function: JUP, OCLN, DSP, CLDN1, DSC1, CLDN4, DSC2, PCDH1, TJP1, PKP2 and PPL.

Therefore, the peptides of the present invention (as exemplified by means of Ac-SEQ ID NO: 1- NH₂, Ac-SEQ ID NO: 2- NH₂ and Ac-SEQ ID NO: 3- NH₂) reduce, prevent and/or eliminate signs of skin aging and/or skin imperfections, more precisely, the results of this example demonstrate the usefulness of the peptides of the present invention to improve tight junctions between keratinocytes, improving cohesion between them and, hence, improving firmness of the skin and reducing wrinkles.

**Example 10.** Analysis of gene expression modulation in primary human dermal fibroblasts, adults.

Peptides Ac-SEQ ID NO: 1- NH₂, Ac-SEQ ID NO: 2- NH₂ and Ac-SEQ ID NO: 3- NH₂ were analysed for its capacity to modulate expression of genes related with the production of collagen and the extracellular matrix (see table 6 for the analysed genes).

The peptides used in this example were prepared in accordance with examples 1 to 5.

A stock solution of the peptide was prepared in water at 1 mg/mL. Working solution was freshly prepared at the specified concentration from stock solution in the corresponding supplemented medium.

Untreated cells were used as basal or negative control samples.

RNA (ribonucleic acid) extraction and RT-qPCR (reverse transcription quantitative polymerase chain reaction) were performed. Briefly, HDFa cells were seeded in duplicate (n=2) in 6-well plates at a density of 4 × 10⁵ cells/well and maintained at standard culture conditions (106 Medium supplemented with 1% (v/v) LSGS; 37°C, 95% room humidity, 5% CO₂) for 24 hours. Then, cells were treated with peptide Ac-SEQ ID NO: 1-NH₂ at the concentration of 0.01 mg/mL for 24 and 48 hours. Untreated cells were used as basal or negative control.

Cells were finally lysed, and replicates were pooled together for RNA extraction using Qiagen RNeasy Mini kit following manufacturer's instructions. Purified RNAs were used to generate the corresponding cDNAs (complementary deoxyribonucleic acids) by reverse transcription using a commercial kit High capacity cDNA reverse transcription kit (Applied Biosystems) which served as templates for amplification. RT-qPCR was performed with the panel of appropriate TaqMan assay probes for genes shown in table 5 (plus GAPDH -Glyceraldehyde 3-phosphate dehydrogenase- that was used as housekeeping gene) and 2x gene expression Master Mix using StepOne plus Real-Time PCR instrument. Amplification included 40 cycles of: 15 seconds at 95°C (denaturation) and 1 minute at 60°C (Annealing and extension) (Arya, M., Shergill, I.S., Williamson, M., Gommersall, L., Arya, N., Patel, H.R. (2005) Basic principles of real-time quantitative PCR. Expert Rev. Mol. Diagn.; 5(2):209-19; and Jozefczuk, J. and Adjaye, J. (2011) Quantitative real-time PCR-based analysis of gene expression. Methods Enzymol. 500; 99-109).

**Table 6. Genes analysed in example 10 (abbreviation and complete name).**

| Abbreviation | Gene |
|---|---|
| COL3A1 | Collagen Type III Alpha 1 Chain |
| MMP1 | Matrix Metalloproteinase 1 |
| MMP3 | Matrix Metalloproteinase 3 |
| FBN1 | Fibrillin 1 |
| TIMP2 | Tissue Inhibitor of Metalloproteinases 2 |
| LAMA1 | Laminin Subunit Alpha 1 |

The obtained data was analysed using the ΔΔCt method, which provides the target gene expression values as fold changes in the treated sample compared with an untreated basal sample. Both samples were normalized with the relative expression of a housekeeping gene GAPDH (Glyceraldehyde 3-phosphate dehydrogenase).

The steps for analysis included:
1. Calculate the average Ct for each condition
2. Calculate the ΔCT test sample and the ΔCT untreated sample
3. Calculate the ΔΔCT: ΔΔCT = ΔCT test sample - ΔCT untreated sample
4. Obtain ratio by 2^{- ΔΔCT}

The results of this assay appear summarized in tables 7 to 10.

**Table 7. Fold change between treated cells and basal control obtained in HDFa cells treated with 0.01 mg/mL of Ac-SEQ ID NO: 1-NH₂ for 24 hours.**

| Abbreviation | Fold change |
|---|---|
| COL3A1 | 1.32 |
| TIMP2 | 1.35 |
| LAMA1 | 1.16 |

**Table 8. Fold change between treated cells and basal control obtained in HDFa cells treated with 0.01 mg/mL of Ac-SEQ ID NO: 1-NH₂ for 48 hours.**

| Abbreviation | Fold change |
|---|---|
| MMP1 | -1.30 |
| MMP3 | -1.69 |

**Table 9. Fold change between treated cells and basal control obtained in HDFa cells treated with 0.01 mg/mL of Ac-SEQ ID NO: 2-NH₂ for 24 hours.**

| Abbreviation | Fold change |
|---|---|
| MMP1 | -1.21 |
| FBN1 | 1.20 |

**Table 10. Fold change between treated cells and basal control obtained in HDFa cells treated with 0.01 mg/mL of Ac-SEQ ID NO: 3-NH₂ for 24 hours.**

| Abbreviation | Fold change |
|---|---|
| MMP1 | -1.46 |
| MMP3 | -1.30 |
| FBN1 | 1.29 |

As can be readily derived from tables 7 and 8, for peptide Ac-SEQ ID NO: 1- NH₂:
- At 24 hours: an upregulation of LAMA1, COL3A1 and TIMP2 was observed.
- At 48 hours: a downregulation of MMP1 and MMP3 was observed.

As can be readily derived from table 9, for peptide Ac-SEQ ID NO: 2- NH₂:
- At 24 hours: an upregulation of FBN1 and a downregulation of MMP1 were observed.

As can be readily derived from table 10, for peptide Ac-SEQ ID NO: 3- NH₂:
- At 24 hours: an upregulation of FBN1 and a downregulation of MMP1 and MMP3 were observed.

Therefore, the peptides of the present invention (as exemplified by means of Ac-SEQ ID NO: 1- NH₂, Ac-SEQ ID NO: 2- NH₂ and Ac-SEQ ID NO: 3- NH₂) showed a favorable regulation of genes with regard to the ECM synthesis and maintenance. Therefore, the peptides of the present invention are able to reduce, prevent and/or eliminate signs of skin aging and/or skin imperfections, more precisely, the results of this example demonstrate the usefulness of the peptides of the present invention to improve firmness of the skin and reduce wrinkles.

**Example 11.** Analysis of tyrosinase activity.

Peptides Ac-SEQ ID NO: 1- NH₂, Ac-SEQ ID NO: 2- NH₂, Ac-SEQ ID NO: 3- NH₂ and Ac-Arg-Arg-Gln-Met-Glu-Glu-NH₂ (Ac-SEQ ID NO: 4-NH₂) were analysed for their capacity to modulate tyrosinase activity.

The peptides used in this example were prepared in accordance with examples 1 to 5.

Briefly, different concentrations of Ac-SEQ ID NO: 1-NH₂ (0.05, 0.25, 0.5 and 2.5 mg/mL), Ac-SEQ ID NO: 2-NH₂ (0.05, 0.25, 0.5 and 2.5 mg/mL), Ac-SEQ ID NO: 3-NH₂ (0.05, 0.25, 0.5 and 2.5 mg/mL) and Ac-SEQ ID NO: 4-NH₂ (0.005, 0.025, 0.05, 0.25, 0.5 and 2.5 mg/mL) and the positive control kojic acid (400 and 800 µM) were incubated with recombinant mushroom tyrosinase for 30 minutes before the addition of L-DOPA (L-3,4 dihidroxiphenilalanine, 2.5 mg/mL in PBS). After 2 hours' reaction at room temperature, the absorbance at 450 nm of each well was determined using the microplate reader Multiskan FC (Thermo Fisher Scientific, MA, USA), which is directly proportional to the amount of dopachrome in the reaction mixture.

Absorbance values were normalized with regard to non-treated wells (control) which were stablished as 100%, obtaining, hence, the percentage of activity for each condition tested.

Results obtained in this example appear summarized in Figure 2 (2A to 2D).

As it can be directly derivable from said figure, peptide Ac-SEQ ID NO: 1- NH₂ (Figure 2A) inhibited tyrosinase in all the concentrations tested, but this effect increased in a dose-dependent from 0.25 mg/mL and onwards.

On its side, peptide Ac-SEQ ID NO: 2- NH₂ (Figure 2B) showed inhibition of tyrosinase activity in a dose-dependent manner from 0.05 mg/mL and onwards.

Peptide Ac-SEQ ID NO: 3- NH₂ (Figure 2C) showed a dose-dependent inhibition of tyrosinase activity at concentrations of 0.25 mg/mL and higher.

On its side, peptide Ac-SEQ ID NO: 4- NH₂ (Figure 2D) (a peptide not of the present invention) did not inhibit tyrosinase activity in any of the concentrations tested. On the contrary, from concentration 0.05 mg/mL and onwards it increased tyrosinase activity in a dose-dependent manner.

In the state of the art it is known that dark circles are caused, among others, due to the deposit of melanin and it is also established that inhibition of tyrosinase activity reduces melanin deposits (MI Ryung Roh, Kee Yang Chung: Infraorbital Dark Circles: Definition, Causes, and Treatment Options, Dermatological Surgery, 35:8:August 2009).

These results demonstrate the usefulness of the peptides of the present invention (as exemplified by means of Ac-SEQ ID NO: 1- NH₂, Ac-SEQ ID NO: 2- NH₂ and Ac-SEQ ID NO: 3- NH₂) for the treatment and/or prevention of hyperpigmentation, preferably periorbital hyperchromia in eyes, but not of similar peptides (Ac-SEQ ID NO: 4- NH₂) which instead of inhibiting tyrosinase activity, it maintained or increased said activity.

**Example 12.** Effect of peptide Ac-SEQ ID NO: 1-NH₂ on angiogenesis.

Peptide Ac-SEQ ID NO: 1- NH₂ was analysed for its capacity to modulate blood vessel formation *in vitro.*

Peptide Ac-SEQ ID NO: 1-NH₂ was prepared in accordance with examples 1 to 5.

In this case, a tube formation assay was performed using Human umbilical vein endothelial cells (HUVECs). Briefly, 40µL of geltrex were added to the wells of a 96-well plate and it was left solidifying for 30 minutes. Afterwards 8000 cells were applied to each well together with:
- Complete medium (basal control).
- Complete medium + 20 µM Suramin (inhibitor of tube formation).
- Complete medium + 10 ng/mL IL-33.
- Complete medium + 10 ng/mL IL-33 + different concentrations of peptide Ac-SEQ ID NO: 1-NH₂ (more precisely, 0.01 mg/mL, 0.05 mg/mL or 0.1 mg/mL).

After 16 hours of incubation cells were visualized by means of microscopy and photos were taken at 10x of each of the wells. For each of the wells, the number of meshes and the total area of the meshes was analysed as an indication of vessel formation (this is, of angiogenesis).

Results appear summarized in Figure 3.

As it is readily derivable from said figure, peptide Ac-SEQ ID NO: 1-NH₂ was able to reverse the vessel formation or angiogenesis induced by IL-33, at all the concentrations tested, both, with regard to the number of meshes and with regard to the total area of meshes.

Therefore, the peptides of the present invention, as exemplified by Ac-SEQ ID NO: 1-NH₂, are able to inhibit and/or prevent angiogenesis and, hence, they are useful for the treatment of cosmetic traits related with angiogenesis or excessive blood vessel formation, preferably eyebags and periorbital hyperchromia.

**Example 13.** Analysis of the ability of the peptides of the present invention to protect against lipid peroxidation.

Peptide Ac-SEQ ID NO: 1- NH₂ was analysed for its capacity to protect from lipid peroxidation by means of a Thiobarbituric Acid Reactive Substances (hereinafter, TBARS) assay.

Peptide Ac-SEQ ID NO: 1-NH₂ was prepared in accordance with examples 1 to 5.

Briefly, Trolox (positive control), 0.01, 0.05, 0.1 and 0.5 mg/mL of peptide Ac-SEQ ID NO: 1- NH₂ in presence of small unilamellar vesicles of egg yolk phosphatidylcholine (EYPC), a substrate for lipid peroxidation were incubated for 2 hours at 37°C in the presence of thiobarbituric acid (TBA). A fluorimetric method was used to evaluate the % of lipid peroxidation compared to the negative control (sample only with small unilamellar vesicles of egg yolk phosphatidylcholine).

The results obtained in this example, appear summarized in Figure 4.

As it can be directly derived from said figure, the peptide of the present invention shows anti-oxidant activity at all the concentrations tested.

Therefore, the peptides of the present invention, as exemplified by Ac-SEQ ID NO: 1-NH₂, were able to protect from lipid peroxidation.

As it is known in the state of the art, lipid peroxidation is related with cellular damage and death due to alterations in the plasmatic membrane.

Therefore, the peptides of the present invention, as exemplified by Ac-SEQ ID NO: 1-NH₂, are useful in cosmetics for the prevention and/or treatment of ageing and in medicine for the prevention and/or treatment of diseases related with lipid peroxidation, preferably, atherosclerosis, Inflammatory Bowel Disease, Retinopathy of Prematurity, Borderline Personality Disorder, Asthma, Parkinson's disease and kidney damage.

**Example 14.** Analysis of the ability of the peptides of the present invention to protect against glycation.

Peptide Ac-SEQ ID NO: 1- NH₂ was analysed for its capacity to reduce formation of Advanced glycation end products (AGEs).

Peptide Ac-SEQ ID NO: 1-NH₂ was prepared in accordance with examples 1 to 5.

Briefly, 0.01, 0.05, 0.1 and 0.5 mg/mL of peptide Ac-SEQ ID NO: 1- NH₂ were mixed together with a glycation agent and its corresponding substrate. Samples were incubated for 72 hours at 37°C. A fluorimetric method was used to evaluate the % of protein glycation compared to the negative control (sample only with sugar substrate).

The results obtained in this example, appear summarized in Figure 5.

As it can be directly derived from said figure, the peptide of the present invention was able to reverse almost completely the glycation produced by the glycation agent, at all the concentrations tested.

Therefore, the peptides of the present invention, as exemplified by Ac-SEQ ID NO: 1-NH₂, were able to protect from protein glycation.

In addition, the ability of the peptides of the present invention to protect from protein glycation was also tested *ex vivo.*

Briefly, human skin explants from a 40-year-old woman were kept in survival in BEM culture medium at 37°C in a humid, 5% CO₂ atmosphere. A cosmetic composition in accordance with Example 6 was prepared containing 1% (w/v Ac-SEQ ID NO: 1-NH₂ and without the peptide (placebo). These creams were applied topically on the basis of 2 µL per explant (2 mg/cm²) and spread using a small spatula on day 0 (D0), day 1, day 4, day 6 and day 8. The control explants did not receive any treatment except the renewal of culture medium. To induce glycation, a solution of Methylglyoxal (MG) was incorporated in the BEM culture medium at a final concentration of 500 µM on day 4, day 6 and day 8, on the concerned batches "MG" (all samples, except control untreated explants). Explants were collected at each timepoint and cut in two parts. Half was fixed in buffered formalin solution and half was frozen at -80°C.

After fixation for 24 hours in buffered formalin, the samples were dehydrated and impregnated in paraffin. 5-µm-thick sections were made using a Leica RM 2125 Minot-type microtome, and the sections were mounted on Superfrost^{®} histological glass slides. Carboxy.methyl lysine (CML) immunostaining (CML is an advanced glycation end product (AGE) found on proteins and lipids as a result of oxidative stress and chemical glycation and is used as a marker of advanced glycation) was performed with a monoclonal anti-CML antibody diluted at 1:25 in PBS-BSA 0.3% (w/v) for 1 night at room temperature and revealed by VECTOR^{®} VIP (Vector Laboratories), a substrate of peroxidase.

As can be observed in Figure 6, peptide Ac-SEQ ID NO: 1-NH₂ protected from MG-induced glycation after 9 days of culture, showing a significant decrease in CML signal (-73%) when compared with MG-exposed untreated explants. Placebo-treated explants did not show a significant decrease in CML staining.

As it is known in the state of the art, glycation is one of the endogenous aging mechanisms that occurs spontaneously with time, but also in a pathological manner during diabetes, renal failure, and inflammation. AGEs are highly accumulated in tissues and organs in numerous age-related degenerative diseases. These toxic adducts (glycotoxins) are implicated in cell dysfunction, especially in diabetic patients and older organisms. AGE formation and accumulation in diabetic patients results in vascular alterations leading to diabetic vasculopathy.

Glycation is also related with extracellular matrix damage (damage to collagen and elastic fibers), inflammation, oxidative stress and apoptosis, and, hence, it is related with ageing.

Therefore, the peptides of the present invention, as exemplified by Ac-SEQ ID NO: 1-NH₂, are able to protect from protein glycation and, hence, are useful in cosmetics for the prevention and/or treatment of ageing and in medicine for the prevention and/or treatment of diseases related with glycation, preferably, diabetes (more preferably diabetic vasculopathy), renal failure, inflammation and age-related degenerative diseases.

**Example 15.** Analysis of Fibrillin-1 in *ex vivo* samples.

Peptide Ac-SEQ ID NO: 1- NH₂ was analysed for its capacity to protect the dermal extracellular matrix structure from glycation.

Peptide Ac-SEQ ID NO: 1-NH₂ was prepared in accordance with examples 1 to 5.

Briefly, human skin explants from a 40-year-old woman were kept in survival in BEM culture medium at 37°C in a humid, 5 % CO₂ atmosphere. A placebo cream and a cream containing 1% Ac-SEQ ID NO: 1-NH₂, prepared according to Example 6, were applied topically on the basis of 2 µL per explant (2mg/cm²) and spread using a small spatula on day 0 (D0), day 1, day 4, day 6 and day 8. The control explants did not receive any treatment except the renewal of culture medium. To induce glycation, a solution of Methylglyoxal (MG) was incorporated in the BEM culture medium at a final concentration of 500 µM on day 4, day 6 and day 8, on the concerned batches "MG" (all samples except control untreated samples). Explants were collected at each timepoint and cut in two parts. Half was fixed in buffered formalin solution and half was frozen at -80°C.

Fibrillin 1 immunostaining was performed on frozen sections with a monoclonal anti-fibrillin 1 antibody diluted at 1:500 in PBS, BSA 0.3% (w/v) and Tween 20 at 0.05% (v/v) for 1h at room temperature, with a biotin/streptavidin amplifying system and revealed with FITC. The nuclei were counterstained with propidium iodide.

Data from Figure 7 shows how exposure to MG induces a significant reduction of fibrillin 1 staining in the dermis when compared to the unexposed control at day 9. Peptide Ac-SEQ ID NO: 1-NH₂ was able to protect from this damage, showing a significant increase in fibrillin 1 staining (86%) at day 9 when compared with the MG-exposed untreated control.

As it is known in the state of the art, fibrillin-1 is the major component of oxytalan fibers, that are found along the dermal-epidermal junctions revealing essential functions in maintaining the integrity and the mechanical proprieties of the skin (Oxlund et al., 1980. J Anat. 131: 611-620). In dermo-cosmetic research, fibrillin-1 is usually used as a bio-marker of the structural state of the extracellular matrix of the dermis.

Therefore, the peptides of the present invention (as exemplified by means of Ac-SEQ ID NO: 1- NH₂) reduce, prevent and/or eliminate signs of skin aging and/or skin imperfections, more precisely, the results of this example demonstrate the usefulness of the peptides of the present invention maintain the integrity and the mechanical proprieties of the skin and, hence, improve firmness of the skin and reduce wrinkles.

**Example 16.** Analysis of the expressions of genes related with the response to sleep deprivation.

Peptide Ac-SEQ ID NO: 1- NH₂ was analysed for its capacity to modulate expression of genes related with the consequences of sleep deprivation (see table 11 for the analysed genes) in HDFa cells, HEKa cells and HUVECs.

The peptide used in this example was prepared in accordance with examples 1 to 5.

A stock solution of the peptide was prepared in water at 1 mg/mL. Working solution was freshly prepared at the specified concentration from stock solution in the corresponding supplemented medium.

Untreated cells were used as basal or negative control samples.

RNA (ribonucleic acid) extraction and RT-qPCR (reverse transcription quantitative polymerase chain reaction) were performed. Briefly, HEKa, HDFa or HUVEC cells were seeded in duplicate (n=2) in 6-well plates at a density of 4 × 10⁵ cells/well (2 × 10⁵ cells/well for HUVEC cells) and maintained at standard culture conditions (106 Medium supplemented with 1% (v/v) LSGS for HDFa cells; Medium Epilife supplemented with 1% (v/v) EDGS and 1% (v/v) penicillin/streptomycin for HEKa cells; Endothelial Cell Growth Medium for HUVEC cells; 37°C, 95% room humidity, 5% CO₂) for 24 hours.

Then, cells were treated with peptide Ac-SEQ ID NO: 1-NH₂ at the concentration of 0.01 mg/mL for 24 hours. Untreated cells were used as basal or negative control.

Cells were finally lysed, and replicates were pooled together for RNA extraction using Qiagen RNeasy Mini kit following manufacturer's instructions. Purified RNAs were used to generate the corresponding cDNAs (complementary deoxyribonucleic acids) by reverse transcription using a commercial kit High capacity cDNA reverse transcription kit (Applied Biosystems) which served as templates for amplification. RT-qPCR was performed with the panel of appropriate TaqMan assay probes for genes shown in table 10 (plus GAPDH -Glyceraldehyde 3-phosphate dehydrogenase- that was used as housekeeping gene) and 2x gene expression Master Mix using StepOne plus Real-Time PCR instrument. Amplification included 40 cycles of: 15 seconds at 95°C (denaturation) and 1 minute at 60°C (Annealing and extension) (Arya, M., Shergill, I.S., Williamson, M., Gommersall, L., Arya, N., Patel, H.R. (2005) Basic principles of real-time quantitative PCR. Expert Rev. Mol. Diagn.; 5(2):209-19; and Jozefczuk, J. and Adjaye, J. (2011) Quantitative real-time PCR-based analysis of gene expression. Methods Enzymol. 500; 99-109).

**Table 11. Genes analysed in example 16 (abbreviation and complete name).**

| Abbreviation | Gene |
|---|---|
| IL1RL1 | Interleukin 1 Receptor Like 1 (IL33 receptor) |
| MTNR1A | Melatonin Receptor 1A |
| SIGIRR | Single Ig IL-1 related receptor (IL33 binding inhibitor) |
| SLC2A1 | Glucose Transporter Type 1 (GLUT1) |
| CXCL8 | Interleukin-8 |
| NFKB1 | Nuclear Factor Kappa B Subunit 1 |

The obtained data was analysed using the ΔΔCt method, which provides the target gene expression values as fold changes in the treated sample compared with an untreated basal sample. Both samples were normalized with the relative expression of a housekeeping gene GAPDH (Glyceraldehyde 3-phosphate dehydrogenase).

The steps for analysis included:
1. Calculate the average Ct for each condition
2. Calculate the ΔCT test sample and the ΔCT untreated sample
3. Calculate the ΔΔCT: ΔΔCT = ΔCT test sample - ΔCT untreated sample
4. Obtain ratio by 2^{- ΔΔCT}

The results of this assay appear also summarized in tables 12 to 14.

**Table 12. Fold change between treated cells and basal control obtained in HDFa cells treated with 0.01 mg/mL of Ac-SEQ ID NO: 1-NH₂ for 24 hours.**

| Abbreviation | Fold change |
|---|---|
| IL1RL1 | -1.26 |

**Table 13. Fold change between treated cells and basal control obtained in HEKa cells treated with 0.01 mg/mL of Ac-SEQ ID NO: 1-NH₂ for 24 hours.**

| Abbreviation | Fold change |
|---|---|
| MTNR1A | 2.11 |
| SIGIRR | 1.27 |
| SLC2A1 | -1.21 |
| CXCL8 | -1.33 |

**Table 14. Fold change between treated cells and basal control obtained in HUVECs treated with 0.01 mg/mL of Ac-SEQ ID NO: 1-NH₂ for 24 hours.**

| Abbreviation | Fold change |
|---|---|
| NFKB1 | -1.24 |

Therefore, the peptides of the present invention (as exemplified by means of Ac-SEQ ID NO: 1- NH₂) reduce, prevent and/or eliminate signs of skin aging, and/or eliminate skin imperfections related with sleep deprivation. This is so because peptide Ac-SEQ ID NO: 1-NH₂, besides its effects on IL-33 levels (ELISA) is able to modulate its signalling by inducing a downregulation of IL33 receptor (IL1RL1) and the IL33-related inhibitor (SIGIRR). Regulation of other inflammatory-related genes (CXCL8 and NFKB1) has also been observed.

Apart from this effect on inflammation, peptide Ac-SEQ ID NO: 1-NH₂ is also able to downregulate the expression of glucose transporter SLC2A1 and upregulate the expression of melatonin receptor MNTR1, both involved in the physiological response to sleep deprivation.

### Example 17. Analysis of gene expression induced by the treatment with Ac-SEQ ID NO: 4-NH₂)

Gene modulation of peptide Ac-SEQ ID NO: 4- NH₂ was analysed in this example (see table 15 for the analysed genes) in HDFa cells and HEKa cells.

The peptide used in this example was prepared in accordance with examples 1 to 5, making the required adaptations.

A stock solution of the peptide was prepared in water at 1 mg/mL. Working solution was freshly prepared at the specified concentration from stock solution in the corresponding supplemented medium.

Untreated cells were used as basal or negative control samples.

RNA (ribonucleic acid) extraction and RT-qPCR (reverse transcription quantitative polymerase chain reaction) were performed. Briefly, HEKa or HDFa cells were seeded in duplicate (n=2) in 6-well plates at a density of 4 × 10⁵ cells/well and maintained at standard culture conditions (106 Medium supplemented with 1% (v/v) LSGS for HDFa cells; Medium Epilife supplemented with 1% (v/v) EDGS and 1% (v/v) penicillin/streptomycin for HEKa cells; 37°C, 95% room humidity, 5% CO₂) for 24 hours.

Then, cells were treated with peptide Ac-SEQ ID NO: 4-NH₂ at the concentration of 0.01 mg/mL for 24 hours. Untreated cells were used as basal or negative control.

Cells were finally lysed, and replicates were pooled together for RNA extraction using Qiagen RNeasy Mini kit following manufacturer's instructions. Purified RNAs were used to generate the corresponding cDNAs (complementary deoxyribonucleic acids) by reverse transcription using a commercial kit High capacity cDNA reverse transcription kit (Applied Biosystems) which served as templates for amplification. RT-qPCR was performed with the panel of appropriate TaqMan assay probes for genes shown in table 10 (plus GAPDH -Glyceraldehyde 3-phosphate dehydrogenase- that was used as housekeeping gene) and 2x gene expression Master Mix using StepOne plus Real-Time PCR instrument. Amplification included 40 cycles of: 15 seconds at 95°C (denaturation) and 1 minute at 60°C (Annealing and extension) (Arya, M., Shergill, I.S., Williamson, M., Gommersall, L., Arya, N., Patel, H.R. (2005) Basic principles of real-time quantitative PCR. Expert Rev. Mol. Diagn.; 5(2):209-19; and Jozefczuk, J. and Adjaye, J. (2011) Quantitative real-time PCR-based analysis of gene expression. Methods Enzymol. 500; 99-109).

**Table 15. Genes analysed in example 17 (abbreviation and complete name).**

| Abbreviation | Gene |
|---|---|
| COL3A1 | Collagen Type III Alpha 1 Chain |
| TIMP2 | Tissue Inhibitor of Metalloproteinases 2 |
| IL1RL1 | Interleukin 1 Receptor Like 1 (IL33 receptor) |
| OCLN | Occludin |
| SIGIRR | Single Ig IL-1 related receptor (IL33 binding inhibitor) |
| CDSN | Corneodesmosin |
| SLC2A1 | Glucose Transporter Type 1 (GLUT1) |
| TJP1 | Tight Junction Protein 1 |
| IL33 | Interleukin-33 |

The obtained data was analysed using the ΔΔCt method, which provides the target gene expression values as fold changes in the treated sample compared with an untreated basal sample. Both samples were normalized with the relative expression of a housekeeping gene GAPDH (Glyceraldehyde 3-phosphate dehydrogenase).

The steps for analysis included:
1. Calculate the average Ct for each condition
2. Calculate the ΔCT test sample and the ΔCT untreated sample
3. Calculate the ΔΔCT: ΔΔCT = ΔCT test sample - ΔCT untreated sample
4. Obtain ratio by 2^{- ΔΔCT}

The results of this assay appear also summarized in tables 16 and 17.

**Table 16. Fold change between treated cells and basal control obtained in HDFa cells treated with 0.01 mg/mL of Ac-SEQ ID NO: 4-NH₂ for 24 hours.**

| Abbreviation | Fold change |
|---|---|
| COL3A1 | -1.28 |
| TIMP2 | -1.26 |
| IL1RL1 | -1.12 |

**Table 17. Fold change between treated cells and basal control obtained in HEKa cells treated with 0.01 mg/mL of Ac-SEQ ID NO: 4-NH₂ for 24 hours.**

| Abbreviation | Gene |
|---|---|
| OCLN | -1.08 |
| SIGIRR | -1.49 |
| CDSN | -1.04 |
| SLC2A1 | -1.07 |
| TJP1 | -1.04 |
| IL33 | -1.01 |

Therefore, as can be directly derived from the results included in tables 16 and 17, peptide Ac-SEQ ID NO: 4-NH₂, which as noted above is not a peptide of the present invention, but is very similar, does not show the favorable gene regulation seen for the peptides of the present invention, on the contrary, it shows an opposite gene regulation in a group of genes and a lack of effect in the expression in another group of genes.

Hence, Ac-SEQ ID NO:4-NH₂, contrary to what has been demonstrated above for the peptides of the present invention:
- Showed a downregulation of COL3A1 and TIMP2. Therefore, Ac-SEQ ID NO: 4-NH₂ showed an unfavourable regulation of genes with regard to the ECM synthesis and maintenance. Therefore, contrary to the peptides of the present invention, Ac-SEQ ID NO: 4-NH₂ was not able to reduce, prevent and/or eliminate signs of skin aging and/or skin imperfections, more precisely, the results of this example demonstrate that said peptide is not able to improve firmness of the skin and reduce wrinkles.
- Showed a slightly downregulation or a lack of regulation of genes OCLN, CDSN and TJP1, this is, key genes related to tight junctions and barrier function. Therefore, Ac-SEQ ID NO: 4-NH₂, contrary to what has been demonstrated for the peptides for the present invention, is not able to improve tight junctions between keratinocytes, and, hence, cannot improve cohesion between them and, hence, it cannot improve firmness of the skin and reduce wrinkles.
- Also, peptide Ac-SEQ ID NO: 4-NH₂ showed a downregulation of SIGIRR and a slight downregulation or lack of regulation of SLC2A1. Therefore, the peptide was unable to revert the response to sleep deprivation.
- Finally, peptide Ac-SEQ ID NO: 4-NH₂ was not able to regulate or alter the expression of genes IL33 and IL1RL1, therefore, being unable to modulate all the response associated with IL33 and explained above.

## Claims

1. Peptide of formula (I):
R₁-AA₁-AA₂-AA₃-AA₄-AA₅-AA₆-R₂
its isomers, salts, solvates and mixtures thereof,
wherein:
the 6 amino acids are 3 arginines, 1 glutamine, 1 methionine and 1 glutamic acid
and wherein:
- R₁ is selected from H, substituted or unsubstituted non-cyclic aliphatic, substituted or unsubstituted alicyclyl, substituted or unsubstituted heterocyclyl, substituted or unsubstituted heteroarylalkyl, substituted or unsubstituted aryl, substituted or unsubstituted aralkyl and R₅-CO-, wherein R₅ is selected from the group formed by substituted or unsubstituted C₁-C₂₄ alkyl radical, substituted or unsubstituted C₂-C₂₄ alkenyl, substituted or unsubstituted C₂-C₂₄ alkynyl, substituted or unsubstituted C₃-C₂₄ cycloalkyl, substituted or unsubstituted C₅-C₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, substituted or unsubstituted heterocyclyl ring of 3 to 10 members, and substituted or unsubstituted heteroarylalkyl of 2 to 24 carbon atoms and 1 to 3 atoms other than carbon and an alkyl chain of 1 to 6 carbon atoms; and
- R₂ is selected from H, -NR₃R₄- , -OR₃ and -SR₃, wherein R₃ and R₄ are independently selected from H, substituted or unsubstituted non-cyclic aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl.

2. Peptide in accordance with claim 1, **characterized in that** it comprises Arg-Arg within its sequence.

3. Peptide in accordance with claim 1 or claim 2, **characterized in that** it comprises Arg-Glu within its sequence.

4. Peptide in accordance with any one of claims 1 to 3, **characterized in that** it comprises Met-Arg within its sequence.

5. Peptide in accordance with any one of claims 1 to 4, **characterized in that** the sequence of the peptide is:
R₁-Arg-Arg-Gln-Met-Arg-Glu-R₂
R₁-Met-Arg-Arg-Glu-Gln-Arg-R₂
R₁-Arg-Glu-Gln-Met-Arg-Arg-R₂

6. Peptide in accordance with any one of claims 1 to 5, **characterized in that** the sequence of the peptide is:
Ac-Arg-Arg-Gln-Met-Arg-Glu-NH₂
Ac-Met-Arg-Arg-Glu-Gln-Arg-NH₂
Ac-Arg-Glu-Gln-Met-Arg-Arg-NH₂

7. Composition comprising a peptide in accordance with any one of claims 1 to 6.

8. Use as a cosmetic of a peptide in accordance with any one of claims 1 to 6 or of a composition in accordance with claim 7 in a subject in need thereof.

9. Use as a cosmetic in accordance with claim 8, **characterized in that** the use as a cosmetic is for the treatment of signs of skin aging and/or skin imperfections.

10. Use as a cosmetic in accordance with claim 9, **characterized in that** the signs of skin aging and/or skin imperfections are eyebags, eyelid imperfections, wrinkles in the surrounding of the eyes, periorbital hyperchromia or combinations thereof.

11. Use as a cosmetic in accordance with claim 10, **characterized in that** the signs of skin aging and/or skin imperfections are associated with sleep deprivation.

12. Peptide in accordance with any one of claims 1 to 6 or composition in accordance with claim 7 for use as a medicament.

13. Peptide in accordance with any one of claims 1 to 6 or composition in accordance with claim 7 for use in the treatment of extensive sagging eyelid, wherein said extensive sagging eyelid leads to visual field loss, ocular or eyelid irritation and/or headaches.

## Patentansprüche

1. Peptid der Formel (I):
R₁-AA₁-AA₂-AA₃-AA₄-AA₅-AA₆-R₂
seine Isomere, Salze, Solvate und Gemische davon,
wobei:
die 6 Aminosäuren 3 Arginine, 1 Glutamin, 1 Methionin und 1 Glutaminsäure sind
und wobei:
- R₁ ausgewählt ist aus H, substituiertem oder unsubstituiertem nicht-cyclischem Aliphat, substituiertem oder unsubstituiertem Alicyclyl, substituiertem oder unsubstituiertem Heterocyclyl, substituiertem oder unsubstituiertem Heteroarylalkyl, substituiertem oder unsubstituiertem Aryl, substituiertem oder unsubstituiertem Aralkyl und R₅-CO-, wobei R₅ ausgewählt ist aus der Gruppe, bestehend aus substituiertem oder unsubstituiertem C₁-C₂₄-Alkylrest, substituiertem oder unsubstituiertem C₂-C₂₄-Alkenyl, substituiertem oder unsubstituiertem C₂-C₂₄-Alkynyl, substituiertem oder unsubstituiertem C₃-C₂₄-Cycloalkyl, substituiertem oder unsubstituiertem C₅-C₂₄-Cycloalkenyl, substituiertem oder unsubstituiertem C₈-C₂₄-Cycloalkynyl, substituiertem oder unsubstituiertem C₆-C₃₀-Aryl, substituiertem oder unsubstituiertem C₇-C₂₄-Aralkyl, einem substituierten oder unsubstituierten Heterocyclylring mit 3 bis 10 Gliedern und substituiertem oder unsubstituiertem Heteroarylalkyl mit 2 bis 24 Kohlenstoffatomen und 1 bis 3 Nichtkohlenstoffatomen und einer Alkylkette mit 1 bis 6 Kohlenstoffatomen; und
- R₂ ausgewählt ist aus H, -NR₃R₄- , -OR₃ und -SR₃, wobei R₃ und R₄ unabhängig voneinander ausgewählt sind aus H, substituierter oder unsubstituierter nichtcyclischer aliphatischer Gruppe, substituiertem oder unsubstituiertem Alicyclyl, substituiertem oder unsubstituiertem Heterocyclyl, substituiertem oder unsubstituiertem Heteroarylalkyl, substituiertem oder unsubstituiertem Aryl und substituiertem oder unsubstituiertem Aralkyl.

2. Peptid nach Anspruch 1, **dadurch gekennzeichnet, dass** es Arg-Arg in seiner Sequenz umfasst.

3. Peptid nach Anspruch 1 oder 2, **dadurch gekennzeichnet, dass** es Arg-Glu in seiner Sequenz umfasst.

4. Peptid nach einem der Ansprüche 1 bis 3, **dadurch gekennzeichnet, dass** es Met-Arg in seiner Sequenz umfasst.

5. Peptid nach einem der Ansprüche 1 bis 4, **dadurch gekennzeichnet, dass** die Sequenz des Peptids folgende ist:
R₁-Arg-Arg-Gln-Met-Arg-Glu-R₂
R₁-Met-Arg-Arg-Glu-Gln-Arg-R₂
R₁-Arg-Glu-Gln-Met-Arg-Arg-R₂

6. Peptid nach einem der Ansprüche 1 bis 5, **dadurch gekennzeichnet, dass** die Sequenz des Peptids folgende ist:
Ac-Arg-Arg-Gln-Met-Arg-Glu-NH₂
Ac-Met-Arg-Arg-Glu-Gln-Arg-NH₂
Ac-Arg-Glu-Gln-Met-Arg-Arg-NH₂

7. Zusammensetzung, umfassend ein Peptid nach einem der Ansprüche 1 bis 6.

8. Verwendung eines Peptids nach einem der Ansprüche 1 bis 6 oder einer Zusammensetzung nach Anspruch 7 als Kosmetikum bei einer Person, die dessen bedarf.

9. Verwendung als Kosmetikum nach Anspruch 8, **dadurch gekennzeichnet, dass** die Verwendung als Kosmetikum für die Behandlung von Anzeichen von Hautalterung und/oder Hautunreinheiten ist.

10. Verwendung als Kosmetikum nach Anspruch 9, **dadurch gekennzeichnet, dass** die Anzeichen von Hautalterung und/oder Hautunreinheiten Tränensäcke, Augenlidunreinheiten, Falten in der Augenumgebung, periorbitale Hyperchromie oder Kombinationen davon sind.

11. Verwendung als Kosmetikum nach Anspruch 10, **dadurch gekennzeichnet, dass** die Anzeichen von Hautalterung und/oder Hautunreinheiten mit Schlafmangel verknüpft sind.

12. Peptid nach einem der Ansprüche 1 bis 6 oder Zusammensetzung nach Anspruch 7 zur Verwendung als Medikament.

13. Peptid nach einem der Ansprüche 1 bis 6 oder Zusammensetzung nach Anspruch 7 zur Verwendung bei der Behandlung von ausgeprägtem Schlupflid, wobei das ausgeprägte Schlupflid zu Gesichtsfeldverlust, Augen- oder Augenlidreizung und/oder Kopfschmerzen führt.

## Revendications

1. Peptide ayant la formule (I) :
R₁-AA₁-AA₂-AA₃-AA₄- AA₅-AA₆-R₂
ses isomères, sels, solvates et mélanges de ceux-ci,
dans lequel :
les 6 acides aminés sont 3 arginines, 1 glutamine, 1 méthionine et 1 acide glutamique
et dans lequel :
- R₁ est choisi parmi H, groupement aliphatique non cyclique substitué ou non substitué, groupement alicyclique substitué ou non substitué, hétérocyclyle substitué ou non substitué, hétéroarylalkyle substitué ou non substitué, aryle substitué ou non substitué, aralkyle substitué ou non substitué et R₅-CO-, dans lequel R₅ est choisi parmi le groupe formé par radical alkyle C₁-C₂₄ substitué ou non substitué, alcényle C₂-C₂₄ substitué ou non substitué, alcynyle C₂-C₂₄ substitué ou non substitué, cycloalkyle C₃-C₂₄ substitué ou non substitué, cycloalcényle C₅-C₂₄ substitué ou non substitué, cycloalcynyle C₈-C₂₄ substitué ou non substitué, aryle C₆-C₃₀ substitué ou non substitué, aralkyle C₇-C₂₄ substitué ou non substitué, cycle hétérocyclyle substitué ou non substitué de 3 à 10 membres, et hétéroaryl-alkyle substitué ou non substitué de 2 à 24 atomes de carbone et de 1 à 3 atomes autres que le carbone et une chaîne alkyle de 1 à 6 atomes de carbone ; et
- R₂ est choisi parmi H, -NR₃R₄-, -OR₃ et -SR₃, dans lesquels R₃ et R₄ sont choisis indépendamment parmi H, groupement aliphatique non cyclique substitué ou non substitué, alicyclyle substitué ou non substitué, hétérocyclyle substitué ou non substitué, hétéroarylalkyle substitué ou non substitué, aryle substitué ou non substitué, et aralkyle substitué ou non substitué.

2. Peptide selon la revendication 1, **caractérisé en ce qu'**il comprend Arg-Arg dans sa séquence.

3. Peptide selon la revendication 1 ou la revendication 2, **caractérisé en ce qu'**il comprend Arg-Glu dans sa séquence.

4. Peptide selon l'une quelconque des revendications 1 à 3, **caractérisé en ce qu'**il comprend Met-Arg dans sa séquence.

5. Peptide selon l'une quelconque des revendications 1 à 4, **caractérisé en ce que** la séquence du peptide est :
R₁-Arg-Arg-Gln-Met-Arg-Glu-R₂
R₁-Met-Arg-Arg-Glu-Gln-Arg-R₂
R₁-Arg-Glu-Gln-Met-Arg-Arg-R₂.

6. Peptide selon l'une quelconque des revendications 1 à 5, **caractérisé en ce que** la séquence du peptide est :
Ac-Arg-Arg-Gln-Met-Arg-Glu-NH₂
Ac-Met-Arg-Arg-Glu-Gln-Arg-NH₂
Ac-Arg-Glu-Gln-Met-Arg-Arg-NH₂.

7. Composition comprenant un peptide selon l'une quelconque des revendications 1 à 6.

8. Utilisation en tant que cosmétique d'un peptide selon l'une quelconque des revendications 1 à 6 ou d'une composition selon la revendication 7 chez un sujet en ayant besoin.

9. Utilisation en tant que cosmétique selon la revendication 8, **caractérisée en ce que** l'utilisation en tant que cosmétique est pour le traitement de signes de vieillissement cutané et/ou des imperfections cutanées.

10. Utilisation en tant que cosmétique selon la revendication 9, **caractérisée en ce que** les signes de vieillissement cutané et/ou des imperfections cutanées sont les cernes, les imperfections de paupières, les rides autour des yeux, l'hyperchromie periorbitale ou des combinaisons de ceux-ci.

11. Utilisation en tant que cosmétique selon la revendication 10, **caractérisée en ce que** les signes de vieillissement cutané et/ou des imperfections cutanées sont liés à la privation du sommeil.

12. Peptide selon l'une quelconque des revendications 1 à 6 ou composition selon la revendication 7 pour son utilisation en tant que médicament.

13. Peptide selon l'une quelconque des revendications 1 à 6 ou composition selon la revendication 7 pour son utilisation dans le traitement d'affaissement important de la paupière, dans lequel ledit affaissement important de la paupière conduit à une perte de champ visuel, une irritation oculaire ou des paupière et/ou des céphalées.
